# EUROPEAN PATENT APPLICATION

(11) **EP 2 050 827 A2**
(43) Date of publication of application: **22.04.2009**
(21) Application number: 08163922.1
(22) Date of filing: 20.09.2004
(51) Int. Cl.: C12Q 1/68, G01N 33/68

(54) **Differentially Expressed Tumour-specific Polypeptides for use in the Diagnosis and Treatment of Cancer**

(30) Priority: 18.09.2003 EP 03090307; 20.10.2003 US 512078 P
(62) Divisional of application: 04765615.2
(71) Applicant: Genmab A/S, 1253 Copenhagen K (DK)
(72) Inventor: Buschmann, Thomas, 14612 Falkensee (DE); Fotiadis, Nikoleta-Kyriaki, 13357 Berlin (DE); Fuchs, Miriam, 4125 Riehen (CH); Heim, Steffen, 01129 Dresden (DE); Lehnherr-Iliania, Tatiana, 3014 Bern (CH); Lamer, Stephanie, 13158 Berlin (DE); Meuer, Jörn, 35094 Caldern (DE); Rothmann-Cosic, Kirsten, 10177 Berlin (DE); Seibert, Volker, 79539 Lörrach (DE); Perez, Silvia Tortola, 082256 Terrassa (ES); Stedronsky, Katrin, Winnipeg Manitoba R3T 4N1 (CA)
(74) Representative: Ziebig, Marlene

(57) **Abstract**

The invention relates to agents and methods for the diagnosis, prognosis and treatment of cancer. Specifically, the invention relates to the use of nucleic and amino acid sequences encoding transmembrane superfamily member 6 (TM4SF6), synaptophysin-like protein (SYPL), stomatin-like 2 (STOML2), Ras-related GTP-binding protein RAGA), nucleotide-sensitive chloride channel 1A (CLNS1A), prion protein (p27-30) (PRNP), guanine nucleotide binding protein beta 2-like 1 (GNB2L1), guanine nucleotide binding protein 4 (GNG4), integral membrane protein 2B (ITM2B), integral membrane protein 1 (ITM1), transmembrane 9 superfamily member 2 (TM9SF2), opiate receptor-like 1 protein (OPRL1), low density lipoprotein receptor-related protein 4 (LRP4), human glomerular epithelial protein 1 (GLEPP1), toll-like receptor 3 (TLR3), and/or zona pellucida glycoprotein 3A (ZP3) for the diagnosis of both early and late stage non-steroid specific cancers, cancer prognosis, as well as screening for therapeutic agents that regulate the gene expression and/or biological activity of said proteins. This invention further relates to the biological technologies designed to inhibit the gene expression and/or biological activity of said proteins including using agents identified in screening assays described herein, vector delivery of antisense polynucleotide sequences, and antibody targeting of said proteins. In specific embodiments, the proteins are of human origin.

SEQUENZPROTOKOLL

<110> Genmap A/S

<120> Differentially Expressed Tumour-specific Polypeptides
      for use in the Diagnosis and Treatment of Cancer

<130> P224503EP/PCdiv-Zie

<140> 04765615.2 Stammanm.
<141> 2004-09-20

<150> EP 03 090 307.4
<151> 2003-09-18

<150> US 60/512,078
<151> 2003-10-20

<150> PCT/EP2004/010780
<151> 2004-09-20

<160> 8

<170> PatentIn Ver. 2.1

<210> 1
<211> 15
<212> PRT
<213> Peptide Human

<400> 1
Leu Leu Asp Pro Ser Tyr Ala Lys Asn Asn Ile Pro Ile Ile Ala
  1               5                  10                  15

<210> 2
<211> 15
<212> PRT
<213> Peptide Human

<400> 2
Lys Arg Pro Pro Gly Phe Asp Arg Val Arg Asn Ala Glu Ile Gly
  1               5                  10                  15

<210> 3
<211> 15
<212> PRT
<213> Peptide Human

<400> 3
Arg Leu Met Glu Glu Asn Trp Asn Ala Glu Lys Arg Ser Pro Thr
  1               5                  10                  15

<210> 4
<211> 13
<212> PRT
<213> Peptide Human

<400> 4
Leu Asp Arg Arg Gly Asp His Glu Val Glu Gln Trp Ala
  1               5                  10

<210> 5
<211> 15
<212> PRT
<213> Peptide Human

<400> 5
Thr Phe Gly Tyr Pro Phe Arg Leu Asn Glu Ala Ser Phe Gln Pro
1               5                   10                  15

<210> 6
<211> 14
<212> PRT
<213> Peptide Human

<400> 6
Gly His Asn Ile Ile Asp Glu Leu Pro Pro Cys Lys Lys Lys
1               5                   10

<210> 7
<211> 15
<212> PRT
<213> Peptide Human

<400> 7
Asn Phe Cys Asp Glu Glu Lys Lys Ser Asp Glu Cys Lys Ala Glu
1               5                   10                  15

<210> 8
<211> 14
<212> PRT
<213> Peptide Human

<400> 8
Gly Glu Arg Ile Glu Pro Ser Pro Tyr Lys Phe Thr Phe Asn
1               5                   10

## Description

The invention relates to agents and methods for the diagnosis, prognosis and treatment of cancer. Specifically, the invention relates to the use of nucleic and amino acid sequences encoding transmembrane superfamily member 6 (TM4SF6), synaptophysin-like protein (SYPL), stomatin-like 2 (STOML2), Ras-related GTP-binding protein RAGA), nucleotide-sensitive chloride channel 1A (CLNS1A), prion protein (p27-30) (PRNP), guanine nucleotide binding protein beta 2-like 1 (GNB2L1), guanine nucleotides binding protein 4 (GNG4), integral membrane protein 2B (ITM2B), integral membrane protein I (ITM1), transmembrane 9 superfamily member 2 (TM9SF2), opiate receptor-like 1 protein (OPRL1), low density lipoprotein receptor-related protein 4 (LRP4), human glomerular epithelial protein I (GLEPP1), toll-like receptor 3 (TLR3), and/or zona pellucida glycoprotein 3A (ZP3) for the diagnosis of both early and late stage non-steroid specific cancers, cancer prognosis, as well as screening for therapeutic agents that regulate the gene expression and/or biological activity of said proteins. This invention further relates to the biological technologies designed to inhibit the gene expression and/or biological activity of said proteins including using agents identified in screening assays described herein, vector delivery of antisense polynucleotide sequences, and antibody targeting of said proteins. In specific embodiments, the proteins are of human origin.

### BACKGROUND TO THE INVENTION

Despite improved therapies for certain forms of non-steroid dependent cancers, cancer still remains the leading cause of the death worldwide. Early detection of the disease often greatly improves the chances of complete remission, thereby making it the choice focus area of research regarding development of appropriate diagnostic and therapeutic tools. Diagnostic companies around the world invest a great deal of their allocated research funds in developing such early detection tools, with a primary focus on the detection of cancer prior to the development of a substantial tumour. Furthermore, these companies are also developing detection tools for post-operative analysis, in order to provide a method of monitoring any trace of cancer (e.g. either in the form of residual tissue from the primary tumour or of secondary tumours caused by metastasis) in the patient following surgical treatment of a tumour. The latter allows the physician to prescribe an appropriate treatment such as chemotherapy, to supplement the surgical therapy.

In order to detect cancer in a patient, large quantities of non-steroid cancer cells must be present within the cancer site in order for the physician to make an effective diagnosis. In practice, this is not the case. Too small quantities of detectable non-steroid dependent cancer cells limit detection during a physical examination of the cancer site. Furthermore, the cancer site may not be susceptible to direct visual observation leaving the physician with no means of making a clear diagnosis. And in the case of potential secondary tumour development, it is not possible to predict where the tumours are likely to occur, thereby making the detection of secondary tumours difficult by visual observation. In order to overcome these problems, sensitive diagnostic tests based on the detection of cancer-associated proteins, in a patient who has, or is about to develop, cancer cells have been developed and are currently commercially available. Examples of such diagnostic tests include alpha-fetoprotein and teratocarcinoma for the identification of primary liver cancer in humans (IZOTOP, Hungary) detection of gastrointestinal cancers using carcinoembryonic antigen (Abbot/Roche, Switzerland), chorionic gonadotropin for the detection of trophoblasts and germ cell cancers (IZOTOP, Hungary; BioCheck Inc., CA), and prostatic acid phosphatase or prostate specific antigens for prostate carcinomas. Although these markers are being used to detect various cancers, the disadvantage of these markers lies in their ability to detect advanced rather than early stage cancers.

Furthermore, many of the commercially available tests are only applicable to a very narrow range of non-steroid dependent cancer types, meaning that such tests often fail to detect other forms of cancer. Furthermore, the narrow applicability of these tests means that it may be necessary to perform multiple tests on a single patient for such diagnostic purposes. Multiple testing is expensive, and the risk that one of the many tests may produce a false-positive test result is high. Therefore, there is a pertinent need for a single diagnostic test that is capable of not only detecting a small number of cancer cells within a patient, but also capable of detecting these cells in a wide variety of non-steroid dependent cancers such as gastrointestinal cancers.

An ideal marker would be one that is genetically expressed in a variety of transformed cells, as well as specific and universally applicable for the purpose of cancer detection. Additionally, it should be immunogenic to allow the generation of antibodies, which can be used in diagnostics as well as in therapy. A further desirable feature of an ideal marker is its location at the plasma membrane of transformed cells, such that at least part of the polypeptide is accessible by potential diagnostic or therapeutic molecules.

To date, no effective pre-symptomatic clinical signs or biomarkers indicating susceptibility to non-steroid dependent cancers exist, making early detection a high priority in the medical management of the disease. Since current therapeutic strategies for early stages of cancer have a higher cure rate than those for later stage cancer, the survival rate of the patient can be increased through early detection. For this reason, the identification of a molecular marker specific for early oncogenic tissues will assist in the diagnosis as well as prognostic monitoring of a developing cancer. Furthermore, such a molecular marker could also be used to screen a library of molecules or compounds for the purpose of developing an effective therapeutic agent which, when administered at an early stage of cancer development, would provide an effective treatment against malignant disease.

The present invention addresses these issues by providing tumour-specific polynucleotides and polypeptides for the diagnosis, prognosis and/ or therapy of non-steroid dependent cancers. Furthermore, these polypeptides and their corresponding polynucleotide sequences are used to screen for agents that alter their biologically activity and/or gene expression for the purpose of identifying and developing a therapeutic agent for the treatment of non-steroid dependent cancers. The present invention provides the immunogenic membrane proteins synaptophysin-like protein (SYPL), stomatin-like 2 (STOML2), Ras-related GTP-binding protein RAGA), nucleotide-sensitive chloride channel 1A (CLNS1A), prion protein (p27-30) (PRNP), guanine nucleotide binding protein beta 2-like 1 (GNB2L1), guanine nucleotide binding protein 4 (GNG4), integral membrane protein 2B (ITM2B), integral membrane protein 1 (ITM1), transmembrane 9 superfamily member 2 (TM9SF2), transmembrane superfamily member 6 (TM4SF6), opiate receptor-like 1 protein (OPRL1), low density lipoprotein receptor-related protein 4 (LRP4), human glomerular epithelial protein 1 (GLEPP1), toll-like receptor 3 (TLR3), and/or zona pellucida glycoprotein 3A (ZP3), nucleic (X68194, AF190167, BC006433, X91788, M13667, BC000214, U31382, NM_021999, L38961, U81006, AF043906, U30185, AB011540, U20489, U88879, X56777, respectively) and amino acid sequences (CAA48279, AAF091421, AAH06433, CAA62902, AAA19664, AAH00214, AAC50204, NP_068839, P46977, AAB38973, ACC64257, AAA84913, BAA32468, AAA82892, AAC34134, respectively) encoding said proteins, derivatives and/or fragments thereof.

Although said immunogenic membrane proteins are not known to be involved in cancer associated processes like for example differentiation, cell growth or apoptosis, they were surprisingly found to be highly expressed on the membranes of transformed cells. Thus, said immunogenic membrane proteins allow the specific identification and targeting of transformed cells.

### SUMMARY OF THE INVENTION

Generally, the present invention relates to the use of the polynucleotide sequences of immunogenic membrane proteins selected from the group consisting of the transmembrane superfamily member 6 (TM4SF6), synaptophysin-like protein (SYPL), stomatin-like 2 (STOML2), Ras-related GTP-binding protein RagA), nucleotide-sensitive chloride channel 1A (CLNS1A), prion protein (p27-30) (PRNP), guanine nucleotide binding protein beta 2-like 1 (GNB2L1), guanine nucleotide binding protein 4 (GNG4), integral membrane protein 2B (ITM2B), integral membrane protein 1 (ITMI), transmembrane 9 superfamily member 2 (TM9SF2), opiate receptor-like 1 protein (OPRL1), low density lipoprotein receptor-related protein 4 (LRP4), human glomerular epithelial protein 1 (GLEPP1), toll-like receptor 3 (TLR3), and/or zona pellucida glycoprotein 3A (ZP3), (X68194, AF190167, BC006433, X91788, M13667, BC000214, U31382, NM_021999, L38961, U81006, AF043906, U30185, AB011540, U20489, U88879, X56777, respectively) genes, the amino acid sequence of the encoded proteins (CAA48279, AAF091421, AAH06433, CAA62902, AAA19664, AAH00214, AAC50204, NP_068839, P46977, AAB38973, ACC64257, AAA84913, BAA32468, AAA82892, AAC34134, respectively), derivatives or fragments thereof, for the diagnosis, prognosis and treatment of non-steroid dependent cancers. In specific embodiments, the given proteins are of human origin.

The invention provides a method of screening for a therapeutic agent for the treatment of a non-steroid dependent cancer resulting from the aberrant expression and/or from an altered biological activity of at least one immunogenic membrane protein selected from the group consisting of TM4SF6, SYPL, STOML2, RAGA, CLNS1A, PRNP, GNB2L1, GNG4, ITM2B, ITM1, TM9SF2, OPRL1, LRP4, GLEPP1, TLR3, or ZP3 polypeptide and/or fragment(s) thereof.

Specifically, the present invention relates to a method for screening a library of test molecules or compounds to identify at least one therapeutic molecule or compound which specifically regulates the expression of said at least one immunogenic membrane protein encoding polynucleotide sequence comprising, contacting a reporter construct under the control of a promoter of said immunogenic membrane gene with a test molecule or compound, or a library of test molecules or compounds, under conditions that allow for specific binding and/or interaction, and detecting the level of expression of the reporter construct (see also Table 1). An alteration in the level of expression relative to a control indicates a potential therapeutic activity.

**Table 1. List of upregulated genes encoding membrane proteins in tumourogenic tissue.**

| **Protein name** | **Abbr.** | **Protein Acc. number** | **Nucleotide Acc. number** |
|---|---|---|---|
| synaptophysin-like protein | SYPL | CAA48279 | X68194 |
| stomatin-like 2 | STOML2 | AAF091421 | AF190167 |
| Ras-related GTP-binding protein | RAGA | AAH06433 | AAH06433 |
| nucleotide-sensitive chloride channel 1A | CLNS1A | CAA62902 | X91788 |
| prion protein (p27-30) | PRNP | AAA19664 | M13667 |
| guanine nucleotide binding protein beta 2-like 1 | GNB2L1 | AAH00214 | HC000214 |
| guanine nucleotide binding protein 4 | GNG4 | AAC50204 | U31382 |
| integral membrane protein 2B | ITM2B | NP_068839 | NM_021999 |
| integral membrane protein 1 | ITM1 | P46977 | L38961 |
| transmembrane 9 superfamily member 2 | TM9SF2 | AAB38973 | U81006 |
| transmembrane superfamily member 6 | TM4SF6 | ACC64257 | AF043906 |
| opiate receptor-like 1 protein | OPRL1 | AAA84913 | U30185 |
| low density lipoprotein receptor-related protein 4 | LRP4 | BAA32468 | AB011540 |
| human glomerular epithelial protein 1 | GLEPP1 | AAA82892 | U20489 |
| toll-like receptor 3 | TLR3 | AAC34134 | U88879 |
| zona pellucida glycoprotein 3A | ZP3 | CAA40095 | X56777 |

The present invention also relates to a method for screening a library of test molecules or compounds to identify at least one therapeutic molecule or compound which specifically binds to and/or interacts with said at least one immunogenic membrane protein (see also Table 1) comprising, contacting said at least one immunogenic membrane protein, derivative or fragment thereof with a test molecule or compound, or a library of test molecules or compounds, under conditions that allow for specific binding and/or interaction, and detecting the level of specific binding and/or interaction. An alteration in the level of binding and/or interaction relative to a control indicates a potential therapeutic activity.

The library of molecules or compounds is preferably selected from the group consisting of DNA and/or RNA molecules, peptides, agonists, antagonists, antibodies, preferably monoclonal antibodies, immunoglobulins, small molecules, and pharmaceutical agents.

Methods are also provided for treating a non-steroid dependent cancer resulting from an aberrant expression and/or biological activity of said at least one immunogenic membrane polypeptide in a mammalian, preferably a human subject: comprising providing a composition that comprises a therapeutic agent identified to bind and/or interact with said at least one immunogenic membrane protein or modulate the level of expression of a given protein (mRNA level), and administering to a mammalian subject a therapeutically effective amount of said composition. The composition may further contain a pharmaceutically acceptable carrier.

The invention provides an alternative method for treating a non-steroid dependent cancer resulting from the aberrant expression of a polynucleotide sequence encoding a said at least one immunogenic membrane polypeptide comprising administering a therapeutically effective amount of an antisense polynucleotide sequence complementary to a polynucleotide sequence encoding said at least one immunogenic membrane protein to a human subject.

Alternatively, a method provided by the invention for treating a non-steroid dependent cancer resulting from the aberrant biological activity of said at least one immunogenic membrane polypeptide comprises administering a therapeutically effective amount of an antibody specific for said at least one immunogenic membrane polypeptide or fragment thereof to a human subject.

A further aspect of the invention is a method of modulating proliferation, differentiation and/or cell migration of target cells comprising administering test molecule or compound identified in the screening methods of the invention to said target cells. Preferably, said target cells are neoplastic cells.

One embodiment of the invention provides a method for determining whether a subject is at risk of developing or has a non-steroid dependent cancer caused by the aberrant expression and/or biological activity of said at least one immunogenic membrane polypeptide, comprising the means for measuring the level of a polynucleotide sequence encoding said at least one immunogenic membrane protein and/or the level of said protein(s) themselve in a sample of cells of the subject. Within the same embodiment, the invention provides a kit with instructions to use the kit based on the above-mentioned method.

The present invention relates to a method of screening for a therapeutic agent for the treatment of a non-steroid dependent cancer charcterized by the aberrant expression and/or an altered biological activity of at least one immunogenic membrane protein selected from the group consisting of TM4SF6, SYPL, STOML2, RAGA, CLNS1A, PRNP, GNB2L1, GNG4, ITM2B, ITM1, TM9SF2, OPRL1, LRP4, GLEPP1, TLR3, or ZP3 polypeptide and/or fragment(s) thereof.
In a preferred embodiment the method of screening for a therapeutic agent according to the invention comprises a) contacting a reporter construct under the control of a promoter of said immunogenic membrane protein with a test molecule or compound, or a library of test molecules or compounds, under conditions to allow specific binding and/or interaction and b) detecting the level of expression of the reporter construct, wherein an alteration in the level of expression relative to a control indicates a potential therapeutic activity.

In another preferred embodiment of the screening method according to the invention the method comprises a) contacting said at least one immunogenic membrane protein, derivative or fragrnant(s) thereof with a test molecule or compound, or a library of test molecules or compounds, under conditions to allow specific binding and/or interaction and b)detecting the level of specific binding and/or interaction, wherein an alteration in the level of interaction relative to a control indicates a potential therapeutic activity.

In the screening method of the invention the screened therapeutic agent preferably suppresses the expression and/or the biological activity of said at least one immunogenic membrane protein. Preferably the library of test molecules or compounds used in the method according to the invention is selected from the group consisting of DNA and/or RNA molecules, peptides, agonists, antagonists, monoclonal antibodies, immunoglobulins, small molecule drugs, pharmaceutical agents and combinations thereof.

It is another object of the present invention to provide a pharmaceutical composition comprising a therapeutic agent identified by the method of screening according to the invention, or a fragment, derivative or homologue thereof. In a preferred embodiment the pharmaceutical composition further comprises a pharmaceutically acceptable carrier.

The present invention further relates to the use of a therapeutic agent identified by the screening method according to the invention or to the use of a derivative or homologue thereof or of a delivery complex containing and/or expressing said therapeutic agent or a derivative or homologue thereof for the preparation of a pharmaceutical composition for the treatment of a non-steroid dependent cancer. The present invention further relates to the use

It is another object of the present invention to provide a method for the suppression of a polynucleotide sequence encoding said at least one immunogenic membrane protein for the purpose of modulating proliferation and/or differentiation or cell death of target cells comprising a) providing a pharmaceutical composition according to the invention or a composition that comprises a therapeutic agent identified by the method of screening according to the invention and b) contacting a therapeutically effective amount of the composition from a) with said target cells. In a preferred embodiment said target cells are neoplastic cells.

The present invention further relates to a method for treating a non-steroid dependent cancer resulting from the aberrant expression and/or from an altered biological activity of said at least one immunogenic membrane protein comprising administering to a subject a therapeutically effective amount of a pharmaceutical composition according to the invention or or a therapeutic agent identified by the method according to the invention.

In a preferred embodiment of the invention the therapeutic agent used in the method for the suppression of a polynucleotide sequence or in the method for treating a non-steroid dependent cancer is an antisense polynucleotide sequence complementary to said at least one immunogenic membrane protein or a fragment thereof. Preferably the binding of said antisense polynucleotide sequence is effective in altering transcription or translation of an mRNA encoding said at least one immunogenic membrane protein according to the invention or a fragment thereof in a host cell expressing said mRNA. Preferably the antisense polynucleotide sequence is expressed from a viral vector, in particular a vaccinia virus, a retrovirus, an adenovirus, or a combination thereof. In another preferred embodiment the antisense polynucleotide sequence is in a delivery complex. The delivery complex according to the invention is preferably a sterol, a lipid or a virus.

It is another object of the present invention to provide a method for the treatment of non-steroid dependent cancer by expressing said at least one immunogenic membrane protein according to the invention or a fragment thereof comprising the stepps of a) isolating a cell or cells, b) contacting said cell(s) with an antisense molecule as defined above and c) delivering said cell(s) to a subject suffering from non-steroid dependent cancer.

The present invention further relates to the use of an antisense molecule according to the invention or of a cell expressing and/or containing said antisense molecule for the preparation of a pharmaceutical composition for the treatment of a non-steroid dependent cancer. In a preferred embodiment of the method for treatment of non-steroid dependent cander or of the use of an antisense molecule said cell(s) are derived from the subject to be treated.

The present invention further relates to the use of at least one of said immunogenic membrane proteins, fragments, derivatives or homologues thereof, or of a cell containing and/or expressing at least one of said immunogenic membrane proteins or fragments, derivatives or homologues thereof, for the preparation of a pharmaceutical composition for the vaccination of subjects.

The present invention further relates to a method of screening for an agent binding specifically to a polynucleotide encoding said at least one immunogenic membrane polypeptide or to said at least one immunogenic membrane polypeptide comprising a) contacting said at least one immunogenic membrane protein, derivative or fragment(s) thereof with a test molecule or compound, or a library of test molecules or compounds, under conditions to allow specific binding and/or interaction and b) detecting the level of specific binding and/or interaction. In a preferred embodiment of the invention the library of test molecules or compounds is selected from the group consisting of DNA and/or RNA molecules, peptides, agonists, antagonists, monoclonal antibodies, immunoglobulins, small molecules, pharmaceutical agents and combinations thereof.

It is another object of the present invention to provide an agent binding specifically to a polynucleotide encoding said at least one immunogenic membrane polypeptide or to said at least one immunogenic membrane polypeptide according to the invention. Preferably said agent is an antibody, in particular a monoclonal antibody. In a preferred embodiment said agent or said antibody comprise an additional moiety.

The present invention further relates to the use of an agent or an antibody according to the invention for the preparation of a pharmaceutical composition for the treatment, diagnosis or the visualisation of non-steroid dependent cancer.

According to the invention the non-steroid dependant cancer is preferably cancer of the breast, lung, gastrointestinal, prostate, ovar, cervix, endometrium, bladder, skin, and/or other cancers arising from epithelial tissue. More preferred the non-steroid dependant cancer according to the invention is a cancer of the skin, in particular melanoma, or colorectal cancer.

It is a further object of the invention to provide a method for determining whether a subject is at risk of developing or has a non-steroid dependent cancer comprising a) determining the amount of polynucleotides encoding said at least one immunogenic membrane polypeptide or the amount of said immunogenic membrane polypeptide(s) according to the invention in a sample of said subject and b) comparing said polynucleotide or polypeptide amount with a control amount that is representative of a healthy subject.

The present invention further relates to a kit for identifying a subject at risk of developing or has a non-steroid dependent cancer caused by the aberrant expression of a polynucleotide sequence encoding a SYPL, STOML2, RAGA, CLNS1A, PRNP, GNB2L1, GNG4, ITM2B, ITM1, TM9SF2, TM4SF6, OPAL1, LRP4, GLEPP1, TLR3, or ZP3 polypeptide comprising, the means for measuring the level of a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3-encoding polynucleotide sequence (X68194, AF190167, BC006433, X91788, M13667, BC000214, U31382, NM_021999, L38961, U81006, AF043906, U30185, AB011540, U20489, U88879, X56777, respectively) in a sample of cells of said subject and instructions to use the kit.

The present invention further relates to a kit for identifying a subject at risk of developing or has a non-steroid dependent cancer resulting from an altered biological activity of an SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide comprising, the means for measuring the level of a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide in a sample of cells of said subject and instructions to use the kit.
According to the invention the at least one immunogenic membrane peptide is preferably of human origin.

### DESCRIPTION TO THE FIGURES

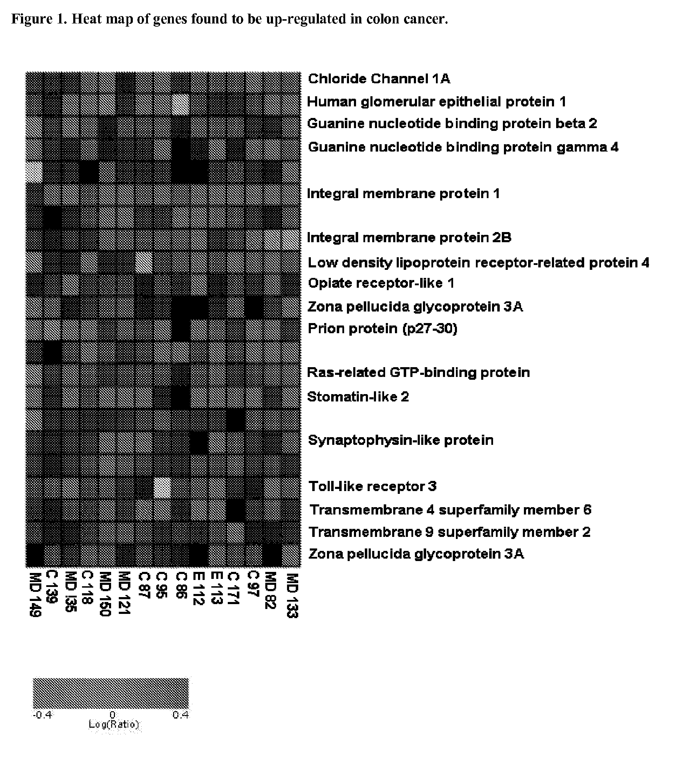

### Figure 1. Heat map of genes found to be up-regulated in colon cancer.

Rows are individual sequences spotted on the used cDNA microarrays, columns are individual samples. Each square in the matrix represents the expression level of a single sequence. Individual genes can be represented on the microarray by more than one sequence. The genes shown are more than 1.5 fold upregulated with a frequency of at least 30% within the 15 experiments mentioned. Red coloured squares indicate an up-regulation of the mentioned gene in the according experiment, whereas green colour indicates a down-regulation. Black colour indicates no regulation or no data for the mentioned sequence in the indicated experiment. The genes Guanine nucleotide binding protein gamma 4, Integral membrane protein 1, Prion protein (p27-30), Stomatin-like 2 and Synaptophysin-like protein are represented with two spots on the cDNA microarrays used therefore two datasets for those genes are shown.

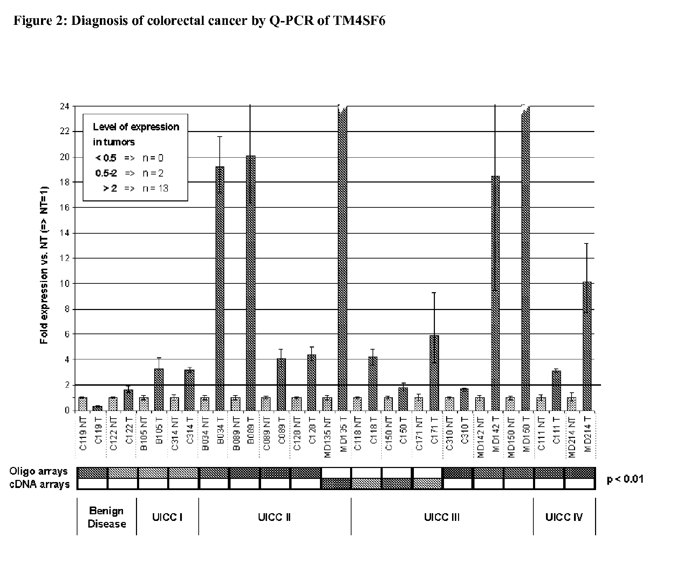

### Figure 2: Diagnosis of colorectal cancer by Q-PCR of TM4SF6

Total RNA from paired non-tumor (blue) and tumor (red) samples was reverse-transcribed in triplicate with random hexamers and then amplified by real-time, quantitative PCR using primers specific for TM4SF6. Amplification and detection were performed using Applied Biosystems' ABI PRISM 7000 Sequence Detection System and SybrGreen as fluorescent dye. Expression of TM4SF6 was normalized to 18S rRNA in every sample. Finally, the fold expression for each tumor was calculated regarding the expression in the corresponding non-tumor tissue (Top). For comparison, the corresponding microarray results are shown (Bottom)

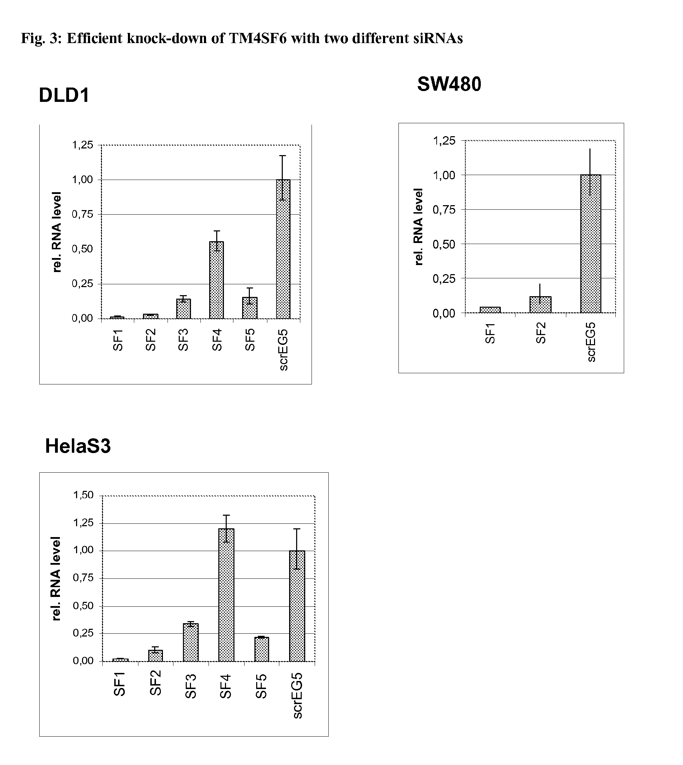

### Figure 3:Efficient knock-down of TM4SF6 with two different siRNAs.

Cells were transfected two or three times with 50nM (DLD1 and HelaS3) or 25nM (SW480) of the indicated siRNAs and were harvested day 4 post first lipofection. RNA was extracted and quantitative PCR (Q-PCR) was performed using specific primers. 18S or Actin was used for normalization. Each column is the mean from at least duplicate Q-PCR reactions.

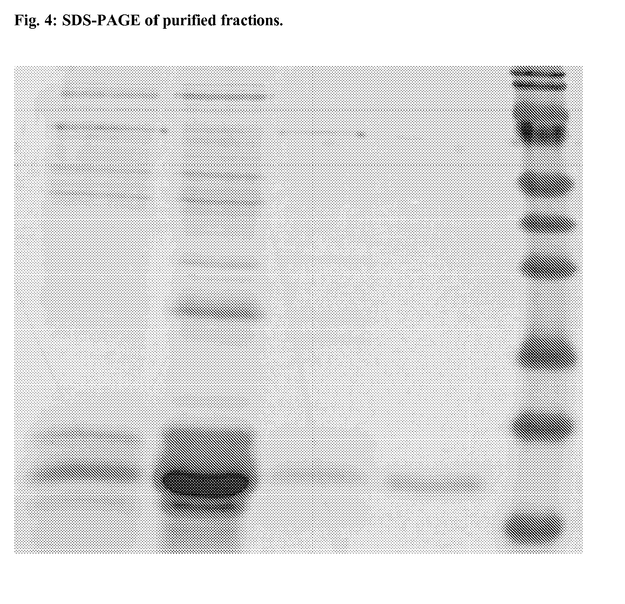

### Figure 4: SDS-PAGE of purified fractions.

The EC2-loop of TM4SF6 was cloned and purified as described in Example 5. Fractions were separated by SDS polyacrylamide gel electrophoresis and the gel stained with coomassie blue. Lane 1: 1st run fraction2, lane 2: 1st run fraction 3, lane 3: 2nd run fraction 3, lane 4: Resource S fraction 28, lane 5: molecular mass standard (sizes from bottom to top: 10, 15, 25, 35, 45, 55, 70, 100, 130, 180 kDa).

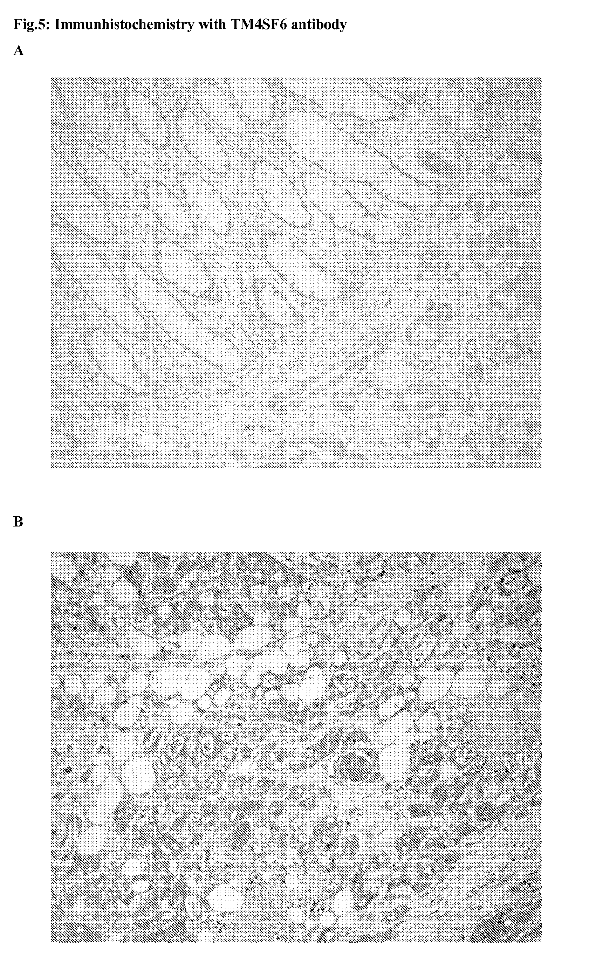

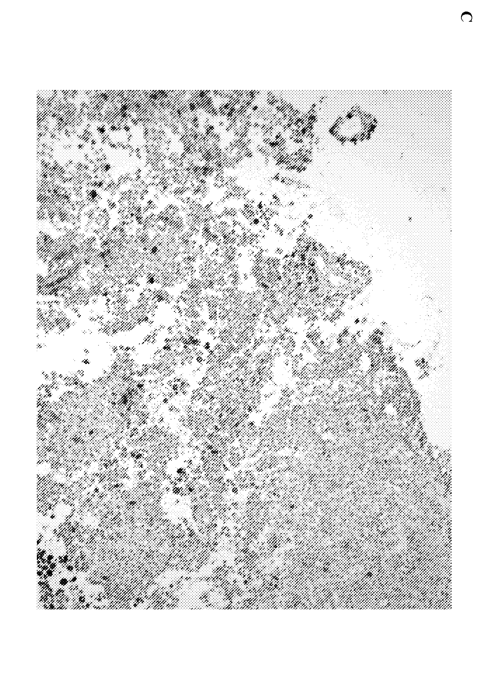

### Figure 5: Immunhistochemistry with TM4SF6 antibody.

Immunhistochemistry was performed using the procedure described in example 6. The TM4SF6 antibody clearly distinguishes between normal colon tissue (Fig. 5A, top left) and neoplastic colon cells (Fig. 5 B, bottom right). While the normal tissue shows no staining, the cancerous cells are positive. The staining is also evident in a colon cancer sample from a different patient (Fig. 5B). TM4SF6 expression was also observed in melanoma cells (Fig.5 C).

### DETAILED DESCRIPTION OF THE INVENTION

It is to be understood that the present invention is not limited to the particular materials and methods described or equipment, as these may vary. It is also to be understood that the terminology used herein is for the purpose of describing particular embodiments only, and is not intended to limit the scope of the present invention, which will be limited only by the appended claims.

It should also be noted that as used herein and in the appended claims, the singular forms "a", "an", and "the" include plural reference unless the context clearly dictates otherwise. Thus, for example, a reference to "a host cell" includes a plethora of such host cells, and a reference to "an antibody" is a reference to one or more antibodies and derivatives thereof known to those skilled in the art, and so forth.

Unless defined otherwise, all technical and scientific terms used herein have the same meanings as commonly understood by one of ordinary skill in the art. Although any materials and methods, or equipment comparable to those specifically described herein can be used to practice or test the present invention, the preferred equipment, materials and methods are described below. All publications mentioned herein are cited for the purpose of describing and disclosing the cell lines, protocols, reagents and vectors which are reported in the publications and which might be used in connection with the invention. Nothing herein is to be construed as an admission that the invention is not entitled to precede such disclosure by virtue of prior invention.

### Definitions

The term 'derivative' refers to a modification of a polypeptide sequence, polynucleotide sequence, or antisense polynucleotide sequence. Polypeptides are modified chemically, and retain a biological activity of an immunogenic membrane protein according to the invention (see Table 1). Furthermore, such modifications may also result in the inhibition of the biologically activity of a given polypeptide. Derivatives of a polypeptide are at least 60% identical or similar to an amino acid sequence encoding said immunogenic membrane protein (see Table 1). Preferred derivatives are at least about 65%, 70%, and even more preferably at least 80%, 85%, 90%, 95%, or 98% identical or similar to an amino acid sequence encoding an immunogenic membrane protein according to the invention (see Table 1).

In a particularly preferred embodiment derivatives having an overall amino acid sequence homology, similarity or identity of at least 65%, at least about 70%, at least about 75%, at least about 80%, at least about 85%, at least about 90%, at least about 95%, at least about 98%, at least about 99% with an amino acid sequence of said immunogenic membrane protein (see Table 1), are used according to the present invention.

Also included under the definition of 'derivative' are modified polynucleotide sequences that differ by one or more nucleotide substitutions, additions or deletions, such as allelic variants; and will, therefore, include sequences that differ from a polynucleotide sequence encoding a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide (see Table 1), due to the degeneracy of the genetic code.
It will be appreciated that the same type of modification may be present in the same or varying degree at several sites in a given polypeptide. Also, a given polypeptide may comprise many types of modifications. Modifications can occur anywhere in a polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, PROTEINS-STRUCTURE AND MOLECULAR PROPERTIES, 2nd Ed., T. E. Creighton, W. H. Freeman and Company, New York (1993) and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York (1983); Polypeptides may be branched or cyclic, with or without branching. Cyclic, branched and branched circular polypeptides may result from post-translational natural processes and may be made by entirely synthetic methods, as well.
Derivatives refer also to conjugates of the polypeptide or fragments thereof to chemical groups or molecule conferring additional properties such as, for example, PEG, fluorescent, radioactive, luminescent or phosphorescent groups, to enzymes, tags, carbohydrate side chains etc.

The term 'fragment' refers to a portion of a polypeptide sequence that comprises at least 5 consecutive amino acid residues and preferably retains the biological activity of an immunogenic membrane protein according to the invention, or to those polynucleotide sequences which, when translated, produce a polypeptide retaining some functional characteristic of said membrane protein, e.g. antigenicity, or structural domain characteristics.

The term 'biological activity' may be used interchangeably with the terms 'biologically active', 'bioactivity' or 'activity' and, for the purposes herein, means an immunogenic/antigenic or effector function that is directly or indirectly, performed by an immunogenic membrane protein according to the invention (whether in its native or denatured conformation), derivative or fragment thereof. Effector functions include phosphorylation (kinase activity) or activation of other molecules, induction of differentiation, mitogenic or growth promoting activity, signal transduction, immune modulation, DNA regulatory functions and the like, whether presently known or inherent. Antigenic functions include possession of an epitope or antigenic site that is capable of cross-reacting with antibodies raised against a naturally occurring or denatured immunogenic membrane protein according to the invention, derivative or fragment thereof. Accordingly, a biological activity of such a protein can be that it functions as an adaptor protein in a signalling pathway of a target cell. Such a signalling pathway can, for example, modulate cell differentiation, proliferation and/or migration of such a cell. A target cell according to the invention can be an epithelial or cancer cell.

The phrases 'nucleotide sequence', 'oligonucleotide sequence' or 'polynucleotide sequence' refer to a nucleotide, oligonucleotide, polynucleotide, or any fragment thereof. These phrases also refer to DNA or RNA of genomic or synthetic origin which may be single-stranded or double-stranded and may represent the sense or the antisense strand, to a peptide polynucleotide sequence (PNA), or to any DNA-like or RNA-like material.

As is well known, genes for a particular polypeptide may exist in single or multiple copies within the genome of an individual. Such duplicate genes may be identical or may have certain modifications, including nucleotide substitution, additions or deletions, all of which still code for polypeptides having substantially the same biological activity. The phrase 'a polynucleotide sequence encoding an immunogenic membrane protein according to the invention may thus refer to one or more genes within a particular individual. Moreover, certain differences in nucleotide sequences may exist between individual organisms, which are called alleles. Such allelic differences may or may not result in differences in the amino acid sequence of the encoded polypeptide yet still encode a protein with the same biological activity.

The term 'a non-steroid dependent cancer' refers to a cancer that arises from epithelial cell origin and may include, but is not limited to, breast, lung, gastrointestinal, prostate, ovarian, cervical, endometrial cancers, bladder, skin cancer, in particular melanoma and/or other cancers. Within the context of the invention epithelial cancers may be of different stages, for example precancerous, early and/or late stage cancers. Cancers may also be of varying degrees in grading, wherein grading refers to the extent of histological differentiation the cancer has progressed. Guidelines to the staging and grading of cancer are known to those skilled in the art, and are described in the 'Cancer Staging Handbook' from the American Joint Committee on Cancer. Also included in the context of the invention, an epithelial cancer may be referred to as a neoplasm of epithelial origin.

The term 'gastrointestinal cancer' refers to a cancer state associated with the gastrointestinal tract of a given subject. In the context of the invention gastrointestinal cancers include, but are not limited to oesophageal, stomach, small intestine, colon, rectal, pancreatic, liver, gallbladder, and biliary tract cancers. Within the context of the invention gastrointestinal cancers may be at different stages, as well as varying degrees of grading (see the 'Cancer Staging Handbook' from the American Joint Committee on Cancer).

The term 'neoplasm' can be used interchangeably with the phrase 'an acute and chronic inflammation of the epithelium' and refers to any new and abnormal growth, specifically a new growth of tissue in which the growth is uncontrolled and progressive.

The terms 'biological sample' and 'test sample' refer to all biological fluids, excretions, tissues and cells isolated from any given subject. In the context of the invention such samples include, but are not limited to, blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract samples.

The phrase 'library of test molecules or compounds' is used herein to include libraries containing DNA molecules, peptides, agonists, antagonists, monoclonal antibodies, immunoglobulins and/or pharmaceutical agents. These may include new or already known molecules or compounds. Furthermore, the terms 'monoclonal antibodies' and 'immunoglobulins' used herein include fragments or derivatives thereof.

'Cells', 'host cells', 'target cells' or 'recombinant host cells' are terms used interchangeably herein, and refer to a cell which has been transformed or transfected, or is capable of transformation or transfection by an exogenous polynucleotide sequence. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A 'delivery complex' shall mean a targeting means (e.g., a molecule that results in higher affinity binding of an antisense polynucleotide sequence, protein, polypeptide, or peptide to a target cell surface and/or increased cellular uptake by a target cell). Examples of targeting means include: sterols (e.g. cholesterol), lipids (e.g. a cationic lipid, virosome or liposome), viruses (e.g. adenovirus, adeno-associated virus, or retrovirus). Preferred complexes are sufficiently stable *in vivo* to prevent significant uncoupling prior to internalization by the target cell. However, the complex is cleavable under appropriate conditions within the cell such that the polynucleotide, protein, polypeptide, or peptide is released in a functional form.

The term 'reporter construct', herein, encompasses a target gene linked in-frame to another sequence to provide a coding unit whose product is easily assayed. Examples of reporter genes include, but are not limited to, β-galactosidase, luciferase, green fluorescent protein (GFP), enhanced green fluorescent protein (EGFP), Ds-Red fluorescent protein, far-red fluorescent protein (He-red), secreted alkaline phosphatase (SEAP), chloramphenicol acetyltransferase (CAT), neomycin etc.

'An antisense polynucleotide sequence' refers to an antisense molecule or anti-gene agent that is comprised of at least about 10 nucleotides. In certain embodiments, an antisense polynucleotide sequence is comprised of at least 15, 18, 20, 25, 30, 35, 40, or 50 nucleotides. Antisense polynucleotide sequences are complementary to the 5' untranslated (UTR) region of the mRNA (up to and including the AUG translation initiation codon), the 3'UTR, or a non-coding region of a given polynucleotide. Binding of an antisense polynucleotide sequence to a polynucleotide sequence encoding a given polypeptide is effective in altering transcription or translation of said mRNA in a host cell expressing said mRNA. Within the context of the invention, antisense polynucleotide sequences may be linear, circular, or triple helix-forming and are complementary to a polynucleotide sequence encoding an immunogenic membrane protein according to the invention (see Table 1). Furthermore, an antisense polynucleotide sequence may be a single or double stranded nucleic acid (e.g., Morpholinos, PNAs or RNAi) and comprise: a) at least one modified base moiety which is selected from the group of 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine,5-methylaminoomethyluracil, 5-methoxyaminomethyl-2-thiouraci beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, 3-(3-amino-3-N-2-carboxypropyl) uracil, (acp3)w, and 2,6-diaminopurine; and/or at least one modified sugar moiety selected from the group including, but not limited to, arabinose, 2-fluoroarabinose, xylulose, and hexose, or lacking a pentose sugar moiety and/or at least one modified phosphate backbone selected from the group consisting of a phosphorothioate, a phosphoramidothioate, a phosphoramidate, a phosphordiamidate, a methylphosphonate, an alkyl phosphotriester, a formacetal or analogue thereof, as well as α-DNA, 2'-O-methyl RNA, a morpholine backbone, repeating N-(2-aminoethyl)-glycine units linked by peptide bonds (peptide nucleic acids; PNAs) or locked nucleic acids (LNAs). As an alternative, the use of RNA interference (RNAi) to inhibit expression of a given protein of known function is also included in the invention. Small interfering RNAs (siRNAs) generated by ribonuclease III cleavage of longer dsRNAs are used to block translation, and are preferably between 20-25 nucleotides in length. Furthermore, the invention also includes the use of RNA Lassos to inhibit the translation of a given protein of known function.

The term 'antagonist' refers, in one embodiment, to a molecule or compound which, when bound to an immunogenic membrane protein according to the invention, decreases the biological activity of such a polypeptide. Such an antagonist may interact with the ligand-binding domain of a protein thereby preventing the ligand-induced activation of the protein. An antagonist may also inhibit the interaction between any one of the above said proteins and another molecule by sterically hindering a critical protein-protein interaction site e.g. a dimerisation domain, a scaffolding domain. Furthermore, an antagonist may decrease and/or inhibit the expression of a polynucleotide sequence encoding said immunogenic membrane protein by interacting with the regulatory region of a given polynucleotide. Alternatively, an antagonist can be a molecule or compound which inhibits or decreases the expression or biological activity of a protein which is located downstream of a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 protein, or which interacts said protein(s). An antagonist can also be a molecule or compound which decreases the amount or the duration of the effect of the biological or immunological activity of any one of the above said proteins. Antagonists may include proteins, polynucleotide sequences, carbohydrates, antibodies or fragments thereof, or any other molecule that exerts these effects.

The terms 'neoplastic cell' or 'neoplastic tissue' refer to a cell or tissue, respectively, that has undergone significant cellular changes (transformation). Such cellular changes are manifested by an escape from specific control mechanisms, increased growth potential, alteration in the cell surface, karyotypic abnormalities, morphological and biochemical deviations from the norm, and other attributes conferring the ability to invade, metastasise and kill.

The term 'antibody' refers to a polypeptide substantially encoded by an immunoglobulin gene or fragments thereof, which bind and recognise a specific antigen. Antibodies exist, for example, as intact immunoglobulins or as a number of well-characterised fragments produced by peptidase digestion. Included within the context of the invention are antibody fragments that are produced either by modifying whole antibodies or synthesised using recombinant DNA methodologies. Within a heterogeneous population of proteins and other biologically active molecules an antibody will, under appropriate binding conditions, interact with its specific antigen or fragment thereof. In this context, the antibodies used within the scope of the invention bind preferentially to a SYPL, STOML2, RAGA, CLNS1A, PRNP, GNB2L1, GNG4, ITM2B, ITM1, TM9SF2, TM4SF6, OPRL1, LRP4, GLEPP1, TLR3, or a ZP3 protein. Also included are antibody conjugates, wherein the antibody or a fragment thereof is coupled to a chemical group or molecule conferring additional properties to the antibody.

### Screening for therapeutics

The invention provides methods for screening for therapeutic agents for the treatment of a non-steroid dependent cancer characterised by aberrant expression and/or biological activity of at least one immunogenic membrane protein selected from the group consisting of a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide and/ fragments thereof..

The methods identify candidates, test molecules or compounds, or agents (e.g. peptides, peptidomimetics, small molecules or other drugs) which decrease and/or inhibit the biological activity of polypeptide, derivative or fragment thereof, or have an inhibitory effect on, for example, the expression of a polynucleotide sequence encoding said polypeptide.

On polynucleotide level such a method comprises for example:
a. contacting a reporter construct under the control of a promoter of said immunogenic membrane protein with a test molecule or compound, or a library of test molecules or compounds, under conditions to allow specific binding and/or interaction; and
b. detecting the level of expression of the reporter construct,
wherein an alteration in the level of expression relative to a control indicates a potential therapeutic activity.
Usually the promoter of a gene is located upstream of the start codon and can be easily identified using well known algorithms such as those provided on the web by the European Bioinformatics Institute.

The level of expression can be measured by methods known in the art, such as, for example, incorporation of radioactive or other labels, enzymatic activity if the reporter is an enzyme or quantitative PCR.. Known reporter molecules include for example β-galactosidase, luciferase, green fluorescent protein (GFP), enhanced green fluorescent protein (EGFP), Ds-Red fluorescent protein, far-red fluorescent protein (He-red), secreted alkaline phosphatase (SEAP), chloramphenicol acetyltransferase (CAT), neomycin etc.

On the protein level, such a method e.g. comprises:
a. contacting said at least one immunogenic membrane protein, derivative or fragment(s) thereof with a test molecule or compound, or a library of test molecules or compounds, under conditions to allow specific binding and/or interaction; and
b. detecting the level of specific binding and/or interaction,
wherein an alteration in the level of interaction relative to a control indicates a potential therapeutic activity.

In one embodiment, the polypeptides have at least one biological activity of an immunogenic membrane protein according to the invention. Proteins or peptides capable of interacting directly or indirectly with such a polypeptide, derivative or fragment thereof, can be identified by various methods. For example, such molecules can be identified using methods based on various binding assays (see references on: yeast-2-hybrid Bemis et al. (1995) Methods Cell Biol. 46, 139-151, Fields and Sternglanz (1994) Trends Genet. 10, 286-292, Topcu and Borden (2000) Pharm. Res. 17, 1049-1055; yeast 3 hybrid: Zhang et al. (1999) Methods Enzymol. 306, 93-113; GST pull-downs as in Palmer et al. (1998) EMBO J. 17, 5037-5047; and phage display as in Scott and Smith (1990) Science 249, 386-390).

In preferred embodiments, the biological activity of a TM4SF6, SYPL, STOML2, RAGA, CLNS1A, PRNP, GNB2L1, GNG4, ITM2B, ITEM1, TM9SF2, OPRL1, LRP4, GLEPP1, TLR3, or ZP3 polypeptide is to modulate cell differentiation, proliferation and/or migration of specific target cells, e.g. epithelial or cancer cells.

In one embodiment, the invention provides assays for screening test molecules or compounds that bind to, interact with, or modulate the biologically active form of said at least one immunogenic membrane protein, derivative or fragment thereof. The test compounds according to the present invention can be obtained using any of the numerous approaches in combinatorial library methods known in the art, including: biological libraries, aptially addressable parallel solid phase or solution phase libraries, synthetic library methods requiring deconvolution, the 'one-bead-one-compound' library method, and synthetic library methods using affinity chromatography selection. The biological library approach is limited to peptide libraries, while the other four approaches are applicable to peptide, non-peptide oligomer or small molecule libraries of compounds (Bindseil et al. (2001) Drug Discov. Today 6, 840-847; Grabley et al. (2000) Ernst Schering Res. Found. Workshop, pp. 217-252; Houghten et al. (2000) Drug Discov. Today 5,276-285; Rader, C. (2001) Drug Discov. Today 6,36-43).

Examples of methods for the synthesis of molecular libraries can be found in the art, for example in: DeWitt et al. (1993) Proc. Natl. Acad. Sci. USA 90, 6909-6913; Erb et al. (1994) Proc. Natl. Acad. Sci. USA 91, 11422-11426; Gallop et al. (1994) J. Med. Chem. 37, 1233-1251; Gordon et al. (1994) J. Med. Chem. 37, 1385-1401.

Libraries of compounds may be presented in solution (e.g., Houghten (1992) Biotechniques 13, 412-421), or on beads (Lam et al. (1991) Nature 354, 82-84), chips (Fodor et al. (1993) Nature 364, 555-556), bacteria (U.S. Patent No. 5,223,409, published June 1993), spores [U.S. Patent Nos. 5,571,698 (published in November 1996); 5,403,484 (published in April 1995); and 5,223,409 (published in June 1993)], plasmids (Cull et al. (1992) Proc. Natl. Acad. Sci. USA 89, 1865-1869) or phages (Scott and Smith (1990) Science. 249, 386-390; Devlin et al. (1990) Science. 249, 404-406; Cwirla et al. (1990) Proc. Natl. Acad. Sci. USA 87, 6378-6382; Felici et al. (1991) J. Mol. Biol. 222, 301-310).

In a preferred embodiment, the library of test molecules or compounds is selected from the group consisting of DNA and/or RNA molecules, peptides, agonists, antagonists, monoclonal antibodies, immunoglobulins, small molecule drugs, pharmaceutical agents and fragments and/or combinations thereof.

According to the invention, the test molecules or compounds identified in the methods of the invention preferably demonstrate the ability to suppress the gene expression and/or the biological activity of at least one immunogenic membrane protein of the invention.

In one embodiment, the assay is a cell-based assay in which a cell expresses a biologically active immunogenic membrane protein, derivative or fragment thereof. The expressed polypeptide is contacted with a test molecule or compound and the ability of the test molecule or compound to bind to, or interact with, the polypeptide is determined. The cell can, for example, be a eukaryotic cell such as, but not limited to a yeast cell, an invertebrate cell (e.g. C. elegans), an insect cell, a teleost cell, a amphibian cell, or a cell of mammalian origin. Determining the ability of the test molecule or compound to bind to, or interact with the polypeptide can be accomplished, for example, by coupling the test molecule or compound with a radioisotope (e.g. ¹²⁵I, ³⁵S, ¹⁴C, or ³H) or enzymatic (e.g. horseradish peroxidase, alkaline phosphatase, or luciferase) label such that binding or interaction of the test molecule or compound to the biologically active polypeptide, derivative or fragment thereof, can be determined by detecting the labelled molecule or compound in the complex. Methods of labelling and detecting interactions of test molecules or compounds with membrane-bound or - associated proteins are known to those skilled in the art.

In a preferred embodiment, the assay comprises contacting a cell, which expresses a biologically active immunogenic membrane protein of the invention, derivative or fragment thereof, with a known molecule or compound which binds, or interacts with said immunogenic membrane protein to form an assay mixture, contacting the assay mixture with a test molecule or compound, and determining the ability of the test molecule or compound to bind to, or interact with a given polypeptide, wherein determining the ability of the test molecule or compound to bind, or interact with, a given polypeptide is compared to a control. The determination of the ability of the test molecule or compound to bind to, or interact with a given immunogenic membrane protein of the invention is based on competitive binding/inhibition kinetics of the test molecule or compound and known target molecules or compounds for a given polypeptide. Methods of detecting competitive binding, or the interaction of two molecules for the same target, are known to those skilled in the art.

In another embodiment, the assay is a cell-based assay comprising contacting a cell expressing a biologically active immunogenic membrane protein of the invention, derivative or fragment thereof, with a test molecule or compound and determining the ability of the test molecule or compound to inhibit the biological activity of a given polypeptide. This can be accomplished, for example, by determining whether said membrane protein continues to bind to or interact with a known target molecule, or whether a specific cellular function (e.g. ion-channelling) has been abrogated. For example, a target molecule can be a component of a signal transduction pathway that facilitates transduction of an extracellular signal, a second intercellular protein that has a catalytic activity, a protein that regulates transcription of specific genes, or a protein that initiates protein translation. Determining the ability of a biologically active immunogenic membrane protein, derivative or fragment thereof, to bind to, or interact with, a target molecule can be accomplished by determining the activity of the target molecule. For example, the activity of the target molecule can be determined by detecting induction of a cellular second messenger of the target [e.g., intracellular Ca²⁺, diacylglycerol and inositol triphosphate IP3)], detecting catalytic/enzymatic activity of the target on an appropriate substrate, detecting the induction (via a regulatory element that may be responsive to a given polypeptide) of a reporter gene operably linked to a polynucleotide encoding a detectable marker, e.g., β-galactosidase, luciferase, green fluorescent protein (GFP), enhanced green fluorescent protein (EGFP), Ds-Red fluorescent protein, far-red fluorescent protein (Hc-red), secreted alkaline phosphatase (SEAP), chloramphenicol acetyltransferase (CAT), neomycin etc, or detecting a cellular response, for example, cellular differentiation, proliferation or migration.

In yet another embodiment, the assay of the present invention is a cell-free assay comprising contacting a biologically active immunogenic membrane protein of the invention, derivative or fragment thereof, with a test molecule or compound, and determining the ability of the test molecule or compound to bind to, or interact with any one of the said polypeptides. Binding or interaction of the test molecule or compound to a given polypeptide can be determined either directly or indirectly as described above. In a preferred embodiment, the assay includes contacting any one of said polypeptides with a known target molecule or compound, which binds, or interacts with a given immunogenic membrane protein to form an assay mixture. The assay mixture is contacted with a test molecule or compound, and the determination of the ability of the test molecule or compound to interact with the polypeptide is based on competitive binding/inhibition kinetics of the test molecule or compound and known molecules or compounds for said polypeptide. Methods of detecting competitive binding, or interaction, of two molecules for the same target, wherein the target is an immunogenic membrane protein, derivative or fragment thereof, are known to those skilled in the art.

In another embodiment, the assay is a cell-free assay comprising contacting said biologically active immunogenic membrane protein, derivative or fragment thereof, with a test molecule or compound, and determining the ability of the test molecule or compound to inhibit the activity of said polypeptide. Determining the ability of the test molecule or compound to inhibit the activity of a given polypeptide can be accomplished, for example, by determining the ability of a the polypeptide to bind to a target molecule by one of the methods described above for determining direct binding. In an alternative embodiment, determining the ability of the test molecule or compound to modulate the activity of said polypeptide can be accomplished by determining the ability of said polypeptide to further modulate a target molecule.

In embodiments of the above assay methods of the present invention, it may be desirable to immobilize either an immunogenic membrane protein of the invention, derivative or fragment thereof, or its target molecule to facilitate separation of complexed from uncomplexed forms of one or both of the proteins, as well as to accommodate automation of the assay. Binding of a test molecule or compound to said immunogenic membrane protein, or interaction of a given immunogenic membrane protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, gluiathiane-S-transferase fusion proteins can be adsorbed onto glutathione sepharose beads (Sigma Chemical; St. Louis, MO) or glutathione derivatized microtiter plates, which are then combined with the test molecule or compound and either the non-adsorbed target protein or a biologically active immunogenic membrane protein, derivative or fragment thereof. The mixture is then incubated under conditions conducive to complex formation (e.g., at physiological conditions for salt and pH). Following incubation, the beads or microtiter plate wells are washed to remove any unbound components and complex formation is measured either directly or indirectly, for example, as described above. Alternatively, the complexes can be dissociated from the matrix, and the level of binding or activity of said polypeptide can be determined using standard techniques.

Other techniques for immobilizing proteins on matrices can also be used in the screening assays of the invention. For example, a biologically active immunogenic membrane protein of the invention, derivative or fragment thereof, or its target molecule can be immobilized utilizing conjugation of biotin and streptavidin.

In another embodiment, inhibitors of expression of said immunogenic membrane protein are identified in a method in which cells are contacted with a candidate molecule or compound and the expression of the selected mRNA or protein [i.e., the mRNA or protein corresponding to a polynucleotide or a biologically active polypeptide] in the cell is determined. In a preferred embodiment, the cell is an animal cell. Even more preferred, the cell can be derived from an insect, fish, amphibian, mouse, rat, or human. The level of expression of the selected mRNA or protein in the presence of the candidate molecule or compound is compared to the level of expression of the selected mRNA or protein in the absence of the candidate molecule or compound. The candidate molecule or compound can then be identified as a inhibitor of expression of a given immunogenic membrane protein of the invention based on this comparison. For example, when expression of the selected mRNA or protein is less (statistically significantly less) in the presence of the candidate molecule or compound than in its absence, the candidate molecule or compound is identified as an inhibitor of the selected mRNA or protein expression. The level of the selected mRNA or protein expression in the cells can be determined by methods described herein.

Those test molecules or compounds identified in the above-described assays are considered within the context of the invention as specific therapeutic agents for an immunogenic membrane protein of the invention.

In another embodiment, said therapeutic agent can also be identified by using a reporter assay, in which the level of expression of a reporter construct, under the control of a promoter of a gene encoding immunogenic membrane protein of the invention, is measured in the presence or absence of a test molecule or compound. A promoter of a gene encoding said immunogenic membrane protein can be isolated by screening a genomic library with a respective cDNA; preferably containing the 5' end of the cDNA. A portion of said promoter, typically from 20 to about 500 base pairs long is then cloned upstream of a reporter gene, e.g., a β-galactosidase, luciferase, green fluorescent protein (GFP), enhanced green fluorescent protein (EGFP), Ds-Red fluorescent protein, far-red fluorescent protein (He-red), secreted alkaline phosphatase (SEAP), chloramphenicol acetyltransferase (CAT), neomycin gene, in a plasmid. This reporter construct is then transfected into cells, e.g., mammalian cells. The transfected cells are distributed into wells of a multi-well plate and various concentrations of test molecules or compounds are added to the wells. After several hours of incubation, the level of expression of the reporter construct is determined according to methods known in the art. A difference in the level of expression of the reporter construct in transfected cells incubated with the test molecule or compound relative to transfected cells incubated without the test molecule or compound will indicate that the test molecule or compound is capable of modulating the expression of a gene encoding said immunogenic membrane protein and is thus a therapeutic agent.

In one embodiment of the invention, said therapeutics can be used for treating a non-steroid dependent cancer, and may be applied to any patient in need of such therapy, including, for example, mammals such as dogs, cats, cows, horses, rabbits, and primates. But most preferably, the patient in need of such therapy is a human.

The present invention also encompasses a method for the suppression of a polynucleotide sequence encoding said at least one immunogenic membrane protein for the purpose of modulating proliferation and/or differentiation or cell death of target cells comprising:
providing a composition that comprises a therapeutic agent identified by a method of the present invention and
contacting a therapeutically effective amount of said composition with said target cells.

A molecule known to bind/interact with said immunogenic membrane protein of the invention can be produced recombinantly (i.e. if the molecule is of amino acid nature), chemically (i.e. nucleic acids, chemical compounds), or biologically synthesised as in the case of antibodies.

This invention further pertains to novel agents identified by the above-described screening assays and uses thereof in the treatment of a non-steroid dependent cancer as described herein.

### Treating a non-steroid dependent cancer

In one embodiment of the invention, a non-steroid dependent cancer resulting from aberrant gene expression and/or biological activity of an immunogenic membrane protein of the invention can be treated with a therapeutic identified by a method of the invention. The aberrant gene expression of a given polynucleotide (see Table 1) may result in altered levels of a biologically active immunogenic membrane protein. Within the context of the invention, increased levels of said membrane protein, as a consequence of aberrant gene expression can result in abnormal cell proliferation, cell differentiation, cell migration, tumour development or metastasis within a given subject. Subjects identified as having a non-steroid dependent cancer or abnormal cell proliferation, cell differentiation, cell migration, tumour development or metastasis can be treated by administering therapeutic of the invention which has been shown to decrease the level of expression of its respective gene, or to inhibit the biological activity of said immunogenic membrane protein.

The invention also provides methods for preventing the formation and/or development of tumours. For example, the development of a tumour can be preceded by the presence of a specific lesion, such as a pre-neoplastic lesion, e.g., hyperplasia, metaplasia, and dysplasia. In the context of the invention such lesions can be found in epithelial tissue. Therefore, the invention provides a method for inhibiting the development of such a lesion into a neoplastic lesion, comprising administering to a subject having a pre-neoplastic lesion, a therapeutic amount of said immunogenic membrane protein of the invention.
This aspect of the invention relates to a method for inducing an immunological response in a mammal which comprises inoculating the mammal with a membrane protein of the invention, or a fragment thereof, adequate to produce antibody and/or T cell immune response to enable the subjects immune system to fight the neoplastic disease. Yet another aspect of the invention relates to a method of inducing immunological response in a mammal which comprises, delivering a immunogenic membrane protein via a vector directing expression of the said protein in vivo in order to induce such an immunological response to produce antibody to protect the subject from diseases.

A further aspect of the invention relates to an immunological/vaccine formulation (composition) which, when introduced into a mammalian host, induces an immunological response in that mammal to a receptor polypeptide wherein the composition comprises an immunogenic membrane protein of the invention or its gene. The vaccine formulation may further comprise a suitable carrier. Since a membrane protein may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.
In the context of the invention, a therapeutic amount of said immunogenic membrane protein of the invention therapeutic will effectively inhibit the development of a pre-neoplastic lesion into a neoplastic lesion with minimal side effects.
Another embodiment of the present invention relates to the use of at least one of said immunogenic membrane proteins, fragments, derivatives or homologues thereof, or of a cell containing and/or expressing at least one of said immunogenic membrane proteins, or fragments, derivatives or homologues thereof, for the preparation of a pharmaceutical composition for the vaccination of subjects.

In a preferred embodiment, the invention provides a method for inhibiting epithelial cell proliferation, differentiation or migration, comprising contacting a tissue in which epithelial cells display an abnormally high proliferative rate, abnormal differentiation and/or migration, such as a during tumour development with a therapeutic agent of the invention The inhibition of the development of a non-steroid dependent cancer is anticipated by the anti-proliferative, anti-differentiating and/or anti-migrative effects of a given therapeutic agent identified within the assays of the invention.

Within the context of the invention, the abnormally proliferating, differentiating or migrating cells are epithelial cells that are present in breast, lung, oesophageal, stomach, small intestinal, colonic, rectal, pancreatic, liver, gallbladder, biliary, prostatic, ovarian, cervical, and endometrial tissues.

Among the approaches which may be used to treat a non-steroid dependent cancer resulting from the aberrant expression of said immunogenic membrane protein of the invention are, for example, using antisense, siRNA, and/or triple helix molecules to inhibit gene expression at the polynucleotide level, or using therapeutic agents identified in the various screening methods provided by the invention. Furthermore, according to the invention antibodies specific for a given polypeptide may also be used to treat a non-steroid dependent cancer. Such antibodies are able to bind specifically to an immunogenic membrane protein of the invention and have substantially greater affinity for the polypeptides of the invention than their affinity for other related polypeptides in the prior art.

Therapeutic agents identified as decreasing/inhibiting said immunogenic membrane protein can be administered to a subject at a therapeutically effective dose to treat a non-steroid dependent cancer.

The invention also provides a pharmaceutical composition comprising a therapeutic agent identified by a method of the invention. An initially identified therapeutic agent may be further optimised by methods known in the art regarding, for example, its activity, toxicity, stability, side effects or physical and/or chemical properties. Therefore, a pharmaceutical composition according to the present invention may contain a fragment, derivative or homologue of an agent identified by the methods of the invention.

Peptides, such as the soluble form of an immunogenic membrane protein, and agonists and antagonist, peptides, antibodies or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. The formulation should suit the mode of administration, and its optimisation is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds. Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.
The dosage range required depends on the choice of molecule, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as are well known in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

Therefore, the invention also provides the use of an therapeutic agent identified by a method according to the invention, or a derivative or homologue thereof, or of a delivery complex, containing and/ or expressing said therapeutic agent or a derivative or homologue thereof, for the preparation of a pharmaceutical composition for the treatment of a non-steroid dependent cancer.

### Diagnostics

Another aspect of the present invention relates to diagnostic assays for determining the expression of an immunogenic membrane protein of the invention, or a polynucleotide encoding a said membrane protein in the context of a biological sample (e.g., blood, serum, cells, tissue) to determine whether an individual is afflicted with a non-steroid dependent cancer, or is at risk of developing a non-steroid dependent cancer resulting from an aberrant expression or biological activity of such an immunogenic membrane protein. The invention also provides for prognostic (or predictive) assays for determining whether an individual is at risk of developing a non-steroid dependent cancer. For example, the level of gene expression can be assayed in a biological sample to determine if a given protein may be present in a biological sample at raised levels as compared to a standard. Such assays can be used for prognostic or predictive purpose to thereby prophylactically treat an individual prior to the development of a non-steroid dependent cancer.

A method for detecting the presence or absence of an immunogenic membrane protein of the invention or a polynucleotide encoding it, in a biological sample involves obtaining a biological sample from a test subject and contacting the biological sample with a compound or agent capable of detecting a given polypeptide or polynucleotide (e.g., mRNA, genomic DNA) such that the presence of a given polypeptide or polynucleotide is directly labelled. Examples include detection of a primary antibody using a fluorescently labelled secondary antibody and end labelling of a DNA probe with biotin such that it can be detected with fluorescently labelled streptavidin. That is, the detection method of the invention can be used to detect mRNA, protein, or genomic DNA in a biological sample *in vitro* as well as *in vivo.* For example, *in vitro* techniques for the detection of mRNA include, but are not limited to, Northern hybridizations, *in situ* hybridizations, reverse transcription-polymerase chain reaction (RT-PCR, see EXAMPLE below) or differential display. *In vitro* techniques for detection of an immunogenic membrane protein include, but are not limited to, enzyme linked immunosorbent assays (ELISAs), Western blots, immuno-precipitations, immunofluorescence, or tissue-array analysis (protein-expression profiling) using antibodies specific for said membrane protein, or fragment thereof. *In vitro* techniques for detection of genomic DNA include Southern hybridizations. Furthermore, *in vivo* techniques for detection of a said immunogenic membrane protein include introducing into a subject a labelled antibody directed against a given polypeptide or fragment thereof.

In one embodiment, the biological sample contains protein molecules from the test subject. Alternatively, the biological sample contains mRNA molecules from the test subject or genomic DNA molecules from the test subject. Preferred biological samples include, but are not limited to, blood, blood serum, plasma, nipple aspirate, urine, semen, seminal fluid, seminal plasma, prostatic fluid, excreta, tears, saliva, sweat, biopsy, ascites, cerebrospinal fluid, milk, lymph, or tissue extract samples isolated by conventional means from a subject.

In another embodiment, the methods further involve obtaining a control biological sample from a control subject, contacting the control sample with a compound or agent capable of detecting said polypeptide, or mRNA, or genomic DNA encoding said polypeptide, such that the presence of the polypeptide, or mRNA, or genomic DNA encoding said polypeptide is detected in the biological sample, and comparing the presence of said polypeptide or mRNA or genomic DNA encoding said polypeptide in the control sample with the presence of the polypeptide or mRNA or genomic DNA encoding the polypeptide in the test sample.

In yet another embodiment, the polynucleotide sequence encoding said immunogenic membrane protein is used as a template for the generation of probes and primers designed for use for identifying and/or cloning a homolog of an immunogenic membrane protein in other cell types, e.g. from other tissues, as well as homologs from other mammalian organisms.

In a preferred embodiment of the present invention, a probe/primer comprising a substantially purified oligonucleotide is used in which the oligonucleotide comprises a region of nucleotide sequence that hybridizes under stringent conditions to at least about 15, preferably about 25, more preferably about 40, 50 or 75 consecutive nucleotides of sense or antisense sequences encoding a said immunogenic membrane protein. For instance, primers based on a polynucleotide sequence encoding said membrane protein can be used in PCR reactions to clone homologs, e.g. specific alleles. Such primers are preferably selected in a region that does not share significant homology to other genes. Likewise, probes based on said sequences can be used to detect transcripts or genomic sequences encoding the same or homologous proteins. In preferred embodiments, the probe further comprises a label group attached thereto and which can be detected, e.g. the label group is selected from amongst radioisotopes, fluorescent compounds, enzymes, and enzyme co-factors.

Furthermore, such probes can also be used as a part of a diagnostic test kit for identifying cells or tissue which over-express an immunogenic membrane protein of the invention, such as by measuring the level of a polynucleotide sequence encoding a given polypeptide in a sample of cells from a patient; e.g. detecting mRNA levels. Briefly, specific nucleotide probes can be generated from said gene (see Table 1) that facilitates histological screening of intact tissue and tissue samples for the presence of an immunogenic membrane protein. The use of probes directed to mRNAs of an immunogenic membrane protein of the invention, or to genomic sequences, can be used for both predictive and therapeutic evaluation, wherein the expression or presence of a given polypeptide manifests, for example, unwanted cell growth, abnormal differentiation or the activation of cell migration within a tissue. Used in conjunction with immunoassays as described herein, the oligonucleotide probes can help facilitate the determination of the molecular basis for cancer associated with the expression of said immunogenic membrane protein. Also within the scope of the invention are kits for determining whether a subject is at risk of developing a non-steroid dependent cancer caused by or contributed by an aberrant expression of an immunogenic membrane protein of the invention (over-expression) and/or biological activity.

In a preferred embodiment, the method can be used for determining whether a subject is at risk of developing a non-steroid dependent cancer. Within the same embodiment, the invention provides a kit with instructions to use the kit based on the above-mentioned method.

### Antisense polynucleotide sequences

A further aspect of the invention relates to 'antisense' therapy. As used herein, 'antisense' therapy refers to the administration or *in situ* generation of antisense polynucleotide sequences or their derivatives which specifically hybridise (e.g. bind) under cellular conditions, with the cellular mRNA and/or genomic DNA encoding an immunogenic membrane protein of the invention so as to inhibit the expression of that protein, e.g. by inhibiting transcription and/or translation. The binding may be by classical base pair complementarity, or, for example, in the case of binding to DNA duplexes, through specific interactions in the major groove of the double helix. In general, 'antisense' therapy refers to the range of techniques generally employed in the art, and includes any therapy that relies on specific binding of the antisense polynucleotide sequence to its target polynucleotide sequences.

An antisense construct of the present invention can be delivered, for example, as an expression plasmid which, when transcribed in the cell, produces RNA which is complementary to at least a unique portion of the cellular mRNA encoding an immunogenic membrane protein of the invention. Alternatively, the antisense construct is nucleic acid molecule that is generated *ex vivo* and which, when introduced into the cell causes inhibition of expression by hybridising with the mRNA and/or genomic sequences of said membrane protein gene. Such antisense polynucleotide sequences are preferably modified such that they are resistant to endogenous nucleases, e.g. exonucleases and/or endonucleases, and are therefore stable *in vivo.* Molecules for use as antisense polynucleotide sequences are selected from the group of, but not limited to phosphodiamidate, phosphoramidate, phosphorothioate and methylphosphonate analogs of DNA (Froehler et al. (1988) Nucleic Acids Res. 16, 4831-4839; Hyrup and Nielsen (1996) Bioorg. Med. Chem. 4, 5-23; Sarin et al. (1988) Proc. Natl. Acad. Sci. USA 85, 7448-7451; Stein et al. (1988) Nucleic Acids Res. 16, 3209-3221; Summerton J. (1999) Biochim. Biophys. Acta 1489, 141-158; Summerton and Weller (1997) Antisense polynucleotide sequences Drug Dev. 7,187-195; Orum and Wengel (2001) Curr. Opin. Mol. Ther. 3, 239-243; Wahlestedt et al. (2000) Proc. Natl. Acad. Sci. USA 97, 5633-5638). Additionally, general approaches to constructing antisense polynucleotide sequences useful for 'antisense' therapy have been reviewed in Letsinger et al. (1989) Proc. Natl. Acad. Sci. USA 86, 6553-6556; Summerton and Weller (1997) Antisense polynucleotide sequences Drug Dev. 7,187-195; Froehler et al. (1988) Nucleic Acids Res. 16,4831-4839.

Antisense approaches involve the design of oligonucleotides (either DNA or RNA, or derivatives thereof) that are complementary to an immunogenic membrane protein of the invention-encoding mRNA. The antisense polynucleotide sequence will bind to mRNA transcripts of said immunogenic membrane protein of the invention and prevent translation. Absolute complementarity, although preferred, is not required. A sequence 'complementary' to a portion of an RNA, as referred to herein, means a sequence having sufficient complementarity to be able to hybridise with the RNA, forming a stable duplex; in the case of double-stranded antisense polynucleotide sequences, a single strand of the duplex DNA may thus be tested, or triplex formation may be assayed. The ability to hybridise will depend on both the degree of complementarity and the length of the antisense polynucleotide sequence. Generally, the longer the hybridising polynucleotide sequence, the more base mismatches with an RNA it may contain and still form a stable duplex (or triplex, as the case may be). One skilled in the art can ascertain a tolerable degree of mismatch by use of standard procedures to determine the melting point of the hybridised complex.

Antisense polynucleotide sequences that are complementary to the 5' end of the mRNA, e.g., the 5' UTR up to and including the AUG translation initiation codon, should work most efficiently at inhibiting translation. However, sequences complementary to the 3' UTR of mRNAs have recently been shown to be effective at inhibiting translation of mRNAs as well (Wagner, R. (1994) Nature 372, 333-335). Therefore, antisense polynucleotide sequences complementary to either the 5' UTR, 3' UTR, or non-coding regions of a gene encoding said immunogenic membrane protein of the invention could be used in an antisense approach to inhibit translation of endogenous mRNA. Antisense polynucleotide sequences that are complementary to the 5' UTR of the mRNA should include the complement of the AUG start codon (e.g. -20 to +20 nucleotides). Antisense polynucleotide sequences complementary to mRNA coding regions are less efficient inhibitors of translation but could be used in accordance with the invention. Whether designed to hybridise to the 5', 3' or non-coding region of a mRNA encoding said at least one immunogenic membrane protein of the invention, antisense polynucleotide sequences should be at least 10 nucleotides in length, and are preferably polynucleotide sequences ranging from 15 to about 50 nucleotides in length. In certain embodiments, the antisense polynucleotide sequence is at least 15 nucleotides, at least 18 nucleotides, at least 20 nucleotides, at least 25 nucleotides, at least 30 nucleotides, at least 35 nucleotides, at least 40 nucleotides, or at least 50 nucleotides in length.

Regardless of the choice of the target sequence, it is preferred that *in vitro* studies are first performed to quantitate the ability of the antisense polynucleotide sequence to inhibit gene expression. It is preferred that these studies utilize controls that distinguish between antisense gene inhibition and non-specific biological effects of antisense polynucleotide sequences. It is also preferred that these studies compare levels of the target RNA or protein with that of an internal control RNA or protein. Additionally, it is envisioned that results obtained using antisense polynucleotide sequences are compared with those obtained using control sequences. It is preferred that the control oligonucleotide sequence is of approximately the same length as the test antisense polynucleotide sequence and should have a similar nucleotide composition, molecular weight and melting temperature. However, these parameters should differ no more than necessary to prevent specific hybridisation to the target sequence. Such sequences may be of sense, inverse or of scrambled nature.

The antisense polynucleotide sequences can be DNA or RNA, or chimeric mixtures, or derivatives thereof, single-stranded or double-stranded, or triple helix-forming oligonucleotides. The antisense polynucleotide sequences can be modified at the base moiety, sugar moiety, or phosphate backbone, for example, to improve stability of the molecule, hybridisation, etc. The antisense polynucleotide sequence may include other appended groups such as peptides (e.g. for targeting host cell receptors *in vivo*), or agents facilitating transport across the cell membrane (see Letsinger et al. (1989) Proc. Natl. Acad. Sci. USA 86, 6553-6556; Lemaitre et al. (1987) Proc. Natl. Acad. Sci. USA 84, 648-652; PCT Publication No. WO 88/09810, published in Dec. 1988) or the blood-brain barrier (see PCT Publication No. WO 89/10134, published in Nov. 1989), hybridisation-triggered cleavage agents (van der Krol et al. (1988) Biotechniques 6, 958-976) or intercalating agents (Zon, G. (1988) Pharm. Res. 5, 539-549). To this end, the antisense polynucleotide sequences may be conjugated to another molecule, e.g., a peptide, hybridisation triggered cross-linking agent, transport agent, triggered cleavage agent, etc. For example, a special delivery system for facilitated morpholino transport using ethoxylated polyethylenimine (EPEI) is described in Morcos et al. U.S. Patent No. 6,228,392 published in May 2001 and Morcos, P.A. (2001) Genesis 30, 94-102.
Preferably, the antisense polynucleotide molecules used according to the invention are selected from RNAi, morpholinos, PNAs, triple-helix forming oligonucleotides, double and single stranded polynucleotide sequences.

The antisense polynucleotide sequence may comprise at least one modified base moiety which is selected from the group including, but not limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-metlryl-2-thiouracil, 2-thiouracil, 4-thiouracil, 5-methyluracil, uracil-5-oxyacetic acid methylester, 3-(3-amino-3-N-2-carboxypropyl) uracil, (acp3)w, and 2,6-diaminopurine.

The antisense polynucleotide sequence may also comprise at least one modified sugar moiety selected from the group including, but not limited to, arabinose, 2-fluoroarabinose, xylulose, and hexose. Furthermore, antisense polynucleotide sequences lacking a pentose sugar moiety are also within the scope of the invention. In yet another embodiment, the antisense polynucleotide sequence comprises at least one modified phosphate backbone selected from the group consisting of a phosphorothioate (Stein and Cohen (1989) In: Oligonucleotides: Antisense Inhibitors of Gene Expression. pp.97-117, J. Cohen, ed. (Boca Raton, FL: CRC Press, Inc.)), a phosphorothioate, a phosphoramidothioate, a phosphoramidate (Froehler et al. (1988) Nucleic Acids Res. 16, 4831-4839), a phosphordiamidate, a methylphosphonate (Miller, P. (1989) In: Oligonucleotides: Antisense Inhibitors of Gene Expression, pp.85-92), an alkyl phosphotriester (Miller, P. (1989) In: Oligonucleotides: Antisense Inhibitors of Gene Expression. pp.82-85), a formacetal or analogue thereof, as well as α-DNA (Rayner et al. (1989) In: Oligonucleotides: Antisense Inhibitors of Gene Expression. pp.119-136, J. Cohen, ed. (Boca Raton, FL: CRC Press, Inc.)), 2'-O-methyl RNA (Shibahara et al. (1989) Nucleic Acids Res. 17, 239-252), a morpholine backbone (Summerton and Weller (1997) Antisense polynucleotide sequences Drug Dev. 7, 187-195), repeating N-(2-aminoethyl)-glycine units linked by peptide bonds (peptide nucleic acids; PNAs) (Hanvey et al. (1992) Science 258, 1481-1485; Nielsen et al. (1993) Anticancer Drug Des. 8, 53-63; Nielsen, P.E. (2000) Curr. Opin. Mol. Ther. 2, 282-287), or locked nucleic acids (LNAs) (Kumar et al. (1998) Bioorg. Med. Chem. Lett. 8, 2219-2222; Petersen et al. (2000) J. Mol. Recognit. 13, 44-53).

Antisense polynucleotides of the invention may be synthesized by standard methods known in the art. As examples, phosphorothioate oligomers may be synthesized by the method of Stein et al. (1988) Nucleic Acids Res. 16, 3209-3021) and methylphosphonate oligomers can be prepared by use of controlled pore glass polymer supports (Sarin et al. (1988) Proc. Natl. Acad. Sci. USA 85, 7448-7451). Morpholino oligomers may be synthesized by the method of Summerton and Weller, U.S. Patent Nos. 5,217,866 (published in June 1993) and 5,185,444 (February 1993), etc.

Furthermore, the use of RNA interference (RNAi) to alter post-transeriptional expression of an immunogenic membrane protein of the invention is also within the scope of the invention (Boutla et al. (2001) Curr. Biol. 11, 1776-1780; Moss, E.G. (2001) Curr. Biol. 11, R772-R775; Bernstein et al. (2001) RNA 7, 1509-1521). RNAi is the process of sequence-specific, post-transcriptional gene silencing, initiated by double-stranded RNA (dsRNA) that is homologous in sequence to the silenced gene. The mediators of sequence-specific messenger RNA degradation are small interfering RNAs (siRNAs) generated by ribonuclease III cleavage from longer dsRNAs. Generally, the length of siRNAs is between 20-25 nucleotides (Elbashir et al. (2001) Nature 411, 494-498).

Triple helix-forming oligonucleotides to modify expression of the gene encoding an immunogenic membrane protein of the invention are also within the scope of the invention. Triple helix formation is used to inhibit the ability of the double helix to open sufficiently for the binding of polymerases, transcription factors, or regulatory molecules. Recent therapeutic advances using triplex DNA have been described in the literature (Gee, J. E. et al. (1994) In: Huber, B. E. and B. I. Carr, Molecular and Immunologic Approaches, Futura Publishing Co., Mt. Kisco, N.Y.).

Furthermore, the invention also includes the use of circular RNA (i.e. RNA Lassos) to inhibit the translation of a specific protein of known function.

Preferred antisense polynucleotides sequences of the above embodiments of the present invention include silencing (RNAi) antisense polynucleotides, morpholino oligomers (Summerton and Weller (1999) Antisense polynucleotide sequences Drug Dev. 7, 187-195), peptide nucleic acids (PNAs) (Egholm et al. (1992) J. Am. Chem. Soc. 114, 1895-1897) and/or locked nucleic acids (LNAs) (Kumar et al. (1998) Bioorg. Med. Chem. Lett. 8, 2219-2222; Petersen et al. (2000) J. Mol. Recognit. 13, 44-53).

In specific embodiments, antisense polynucleotide sequences are delivered to cells that express an immunogenic membrane protein of the invention *in vivo*. A number of methods have been developed for delivering antisense DNA or RNA to cells; e.g., antisense molecules can be injected directly into the tissue site, or modified antisense polynucleotide sequences, designed to target the desired cells e.g., antisense linked to peptides or antibodies that specifically bind receptors or antigens expressed on the target cell surface can be administered systemically.

In a preferred embodiment of the present invention the antisense polynucleotide sequence is expressed from a viral vector. Examples for suitable viruses are, but are not limited to, vaccinia virus, a retrovirus, an adenovirus, or a combination thereof.

The antisense polynucleotide sequence may also be contained in a delivery complex, such as e.g. a sterol, lipid, virus etc. Also a cell may serve as a delivery complex. The invention thus provides a method for the treatment of non-steroid dependent cancer characterised by expressing said at least one immunogenic membrane protein or a fragment thereof comprising the steps of isolating a cell or cells, contacting said cell(s) with an antisense molecule of the invention, and delivering said cell(s) to a subject suffering from non-steroid dependent cancer.

An antisense molecule of the invention or a cell expressing and/or containing said antisense molecule can also be used for the preparation of a pharmaceutical composition for the treatment of a non-steroid dependent cancer. Preferably said cell(s) are derived from the subject to be treated.

### Probes and Primers

Within the context of the invention, polynucleotide sequences encoding a human immunogenic membrane protein of the invention (see Table 1) will further allow the generation of probes and primers designed for use in identifying and/or cloning homologs in other cell types, e.g. from other tissues, as well as immunogenic membrane protein homologs from other mammalian organisms.

Preferred polynucleotide sequences for use as a probe, according to the methods of the invention, include polynucleotide sequences comprising a nucleotide sequence having at least about 15, at least about 30, preferably at least about 50, more preferably at least about 100, and even more preferably at least about 200 consecutive nucleotides from a polynucleotide sequence encoding an immunogenic membrane protein of the invention (see Table 1), or a fragment thereof. In a preferred embodiment, a portion of the polynucleotide sequence corresponds to any segment of the complete coding sequence said membrane protein gene. Alternatively, a portion can be a specific polynucleotide sequence encoding a conserved motif or domain of a human immunogenic membrane protein, e.g. extracellular domain. Alternatively, a portion can be a polynucleotide sequence located between polynucleotide sequences encoding conserved motifs of a human immunogenic membrane protein.

The invention further pertains to polynucleotide sequence molecules for use as probes/primer (i.e. non-coding polynucleotide sequence molecules), which can comprise at least about 15, 18, 20, 25, 30, 40, 50, 100, 125, 150 or 200 nucleotides or base pairs. Yet other preferred polynucleotide sequences comprise at least about 300, at least about 350, at least about 400, at least about 450, at least about 500, or at least about 600 nucleotides of the respective cDNAs. In some embodiments, the polynucleotide sequences of the invention correspond to the 5' portion of a polynucleotide sequence encoding an immunogenic membrane protein of the invention (see Table 1).

Also used in the invention are polynucleotide sequences that are capable of hybridising to a polynucleotide sequence encoding said membrane protein, and in particular, to those shown in Table 1, and fragments thereof, under various conditions of stringency. Conditions that promote hybridisation of polynucleotide sequences are known to those skilled in the art (see Current Protocols in Molecular Biology (1989, John Wiley & Sons, N.Y. 6.3.1-6.3.6; Jowett, T. (2001) Methods 23, 345-358).

In a preferred embodiment, the present invention also provides a probe/primer comprising a substantially purified oligonucleotide, which oligonucleotide comprises a region of a polynucleotide sequence that hybridises under stringent conditions to at least about 15, preferably about 30, more preferably about 50, 100 or 200 consecutive nucleotides of sense or antisense sequence of a polynucleotide sequence encoding an immunogenic membrane protein (see Table 1), or naturally occurring mutants thereof. For instance, primers based a given polynucleotide sequence can be used in PCR reactions to clone a homolog. Such primers are preferably selected in a region that does not share significant homology to other genes. Likewise, probes based on the subject membrane protein -encoding polynucleotide sequences can be used to detect transcripts or genomic sequences encoding the same or homologous proteins. In preferred embodiments, the probe further comprises a label group attached thereto and which can be detected, e.g. the label group is selected from amongst radioisotopes, fluorescent compounds, enzymes, and enzyme co-factors.

Such probes can also be used as a part of a diagnostic test kit for identifying cells or tissue which over-express an immunogenic membrane protein of the invention, such as by measuring the level of a respective polynucleotide sequence (see Table 1) in a sample of cells from a patient; e.g. detecting mRNA levels. The use of probes directed to subject polynucleotide sequences, or to subject genomic sequences, can be used for both predictive and therapeutic evaluation of altered gene expression levels which might be manifested in, for example, unwanted cell growth or abnormal differentiation of tissue. Also within the scope of the invention are kits for determining whether a subject is at risk of developing a non-steroid dependent cancer resulting from over-expression of a polynucleotides sequence encoding an immunogenic membrane protein of the invention. The kits may include probes/primers specific for a given polynucleotide sequence encoding said membrane protein, reaction solutions, and instructions of how to use the kit.

### Vectors

Expression vectors containing a polynucleotide sequence encoding an antagonistic polypeptide, peptide or antisense polynucleotide sequence to an immunogenic membrane protein of the invention or polynucleotide sequence and being operably linked to at least one transcriptional regulatory sequence may be used for practicing the present invention. Regulatory sequences are recognized by those skilled in the art, and are specifically selected to inhibit the expression and/or biologically activity of an endogenous immunogenic membrane protein. Such regulatory sequences are described in Rodriguez and Chamberlin Eds. (1982) Promoters: Structure and function. NY. Praeger; Khoury and Gruss (1983) Cell 33, 313-314. In one embodiment, the expression vector includes a polynucleotide sequence encoding an antisense polynucleotide sequence for the purpose of inhibiting the expression of an immunogenic membrane protein gene. In another embodiment, the expression vector contains a polynucleotide sequence encoding a polypeptide or peptide which can be used to inhibit the biological activity of said immunogenic membrane protein. Such expression vectors can be used as a part of a gene therapy protocol. Thus, another aspect of the invention features expression vectors for *in vivo, in vitro,* or *ex vivo* transfection and expression of an antagonistic polypeptide, peptide or an antisense polynucleotide sequence to abrogate the biological activity of said immunogenic membrane protein (e.g. differentiation of epithelial or cancer cells). This could be desirable, for example, when the naturally occurring form of the protein is over-expressed; or to deliver a form of the protein which alters differentiation of tissue (e.g. inhibiting cell transformation, differentiation or proliferation of cancer cells.

In addition to viral transfer methods, non-viral methods can also be employed to cause expression of an antagonistic peptide or polypeptide in the tissue of an animal. Most non-viral methods of gene transfer rely on normal mechanisms used by mammalian cells for the uptake and intracellular transport of macromolecules. In preferred embodiments, non-viral targeting means rely on endocytic pathways for the uptake of a antagonistic peptide or polypeptide-encoding gene by the targeted cell. Exemplary targeting means of this type include liposomal derived systems, poly-lysine conjugates, and artificial viral envelopes.

### Antibodies

Antibodies generated against the immunogenic membrane proteins of the invention can be obtained by administering the polypeptides or epitope-bearing fragments, analogs or cells to an animal, preferably a nonhuman, using routine protocols and may be of any isotype (IgG, IgA, IgM, IgE, etc). Antibody fragments may be generated by conventional techniques well known in the art, such as the digestion with certain proteases. Thus, the term includes segments of proteolytically-cleaved or recombinantly-prepared portions of an antibody molecule that are capable of selectively reacting with a certain protein. Nonlimiting examples of such proteolytic and/or recombinant fragments include Fab, F(ab').sub.2, Fab', Fv, and single chain antibodies (scFv) containing a V[L] and/or V[H] domain joined by a peptide linker. The scFv's may be covalently or non-covalently linked to form antibodies having two or more binding sites. The present invention includes polyclonal, monoclonal, or other purified preparations of antibodies and recombinant antibodies.

For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor et al., Immunology Today (1983) 4:72) and the EBV-hybridoma technique (Cole et al., MONOCLONAL ANTIBODIES AND CANCER THERAPY, pp. 77-96, Alan R. Liss, Inc., 1985).

Techniques for the production of single chain antibodies (e.g. described in U.S. Pat. No. 4,946,778) can also be adapted to produce single chain antibodies against polypeptides of this invention. Also, transgenic mice, or other organisms including other mammals, may be used to express humanized antibodies.
Antibodies of the present invention may be monospecific, dispecific, trispecific, or of greater multispecificity. Antibodies directed against an immunogenic membrane protein of the present invention and useful for the detection of cancer may be detected with separate antibodies, or may be detected with the same antibody. Alternatively, a multispecific antibody may exhibit different specificities for different epitopes on the same protein. Also encompassed in the present invention are antibodies which bind to polypeptide molecules which are encoded by one or more nucleic acid sequences which are complementary to, or hybridize to the sequences of an immunogenic membrane protein of the invention or one or more sequences which are complementary to, or hybridize to a nucleic acid sequence which encodes an said membrane protein.

Antibodies of the present invention which are useful for the detection of cancer may further act as agonists or antagonists of the activity of the polypeptide molecules to which they bind, and may thus be useful as therapeutic molecules for the treatment or prevention of cancer. Antibodies do not have to be used alone, and can be fused to other polypeptides, including a heterologous polypeptide at the N- or C-terminus of the antibody polypeptide sequence. For example, an antibody useful in the present invention may be fused with a detectable label to facilitate detection of the antibody when bound to a target polypeptide. Methods for detectably labeling an antibody polypeptide are known to those of skill in the art.

For the production of antibodies useful in the present invention, various hosts including goats, rabbits, rats, mice, etc., may be immunized by injection with the protein products (or any portion, fragment, or oligonucleotide thereof which retains immunogenic properties) of the candidate genes of the invention. Depending on the host species, various adjuvants may be used to increase the immunological response. Such adjuvants include but are not limited to Freund's, mineral gels such as aluminum hydroxide, and surface active substances such as lysolecithin, pluronic polyols, polyanions, peptides, oil emulsions, keyhole limpet hemocyanin, and dinitrophenol. BCG (bacilli Calmette-Guerin) and Corynebacterium parvum are potentially useful human adjuvants.

Polyclonal antisera or monoclonal antibodies can be made using methods known in the art. A mammal such as a mouse, hamster, or rabbit, can be immunized with an immunogenic form of a immunogenic membrane protein of the invention, fragment, derivative, or variant form thereof. Techniques for conferring immunogenicity on such molecules include conjugation to carriers or other techniques well known in the art. For example, the immunogenic molecule can be administered in the presence of adjuvant as described above. Immunization can be monitored by detection of antibody titers in plasma or serum. Standard immunoassay procedures can be used with the immunogen as antigen to assess the levels and the specificity of antibodies. Following immunization, antisera can be obtained and, if desired, polyclonal antibodies isolated from the sera.

Chimeric antibodies, i.e., antibody molecules that combine a non-human animal variable region and a human constant region also are within the scope of the invention. Chimeric antibody molecules include, for example, the antigen binding domain from an antibody of a mouse, rat, or other species, with human constant regions. Standard methods may be used to make chimeric antibodies containing the immunoglobulin variable region which recognizes an immunogenic membrane protein of the invention (see, e.g., Morrison et al., 1985, Proc. Natl. Acad. Sci. USA 81: 6851; Takeda et al., 1985, Nature 314: 452; U.S. Pat. No. 4,816,567; U.S. Pat. No. 4,816,397).

Extensive guidance can be found in the literature for covalently linking molecular tags to binding compounds, such as antibodies, e.g. Hermanson, Bioconjugate Techniques, (Academic Press, New York, 1996), and the like. In one aspect of the invention, one or more molecular tags are attached directly or indirectly to common reactive groups on a binding compound. Common reactive groups include amine, thiol, carboxylate, hydroxyl, aldehyde, ketone, and the like, and may be coupled to molecular tags by commercially available cross-linking agents, e.g. Hermanson (cited above); Haugland, Handbook of Fluorescent Probes and Research Products, Ninth Edition (Molecular Probes, Eugene, Oreg., 2002).

As is well understood in the art, antibodies maybe conjugated with labels selected from a group including a chromogen, a catalyst, an enzyme, a fluorophore, a chemiluminescent molecule, biotin and a radioisotope. A large number of enzymes suitable for use as labels is disclosed in U.S. Pat. No. 4,366,241, U.S. Pat. No. 4,843,000, and U.S. Pat. No. 4,849,338, each of which is herein incorporated by reference. Suitable enzyme labels useful in the present invention include alkaline phosphatase, horseradish peroxidase, luciferase, beta-galactosidase, glucose oxidase, lysozyme, malate dehydrogenase and the like. The enzyme label may be used alone or in combination with a second enzyme in solution. Preferably for therapeutic purposes, the antibody is conjugated to a cytotoxic moiety.

Fluorophores may be selected from a group including fluorescein isothiocyanate (FITC), tetramethylrhodamine isothiocyanate (TRITC), allophycocyanin (APC), Texas Red (TR), Cy5 or R-Phycoerythrin (RPE). Examples of useful fluorophores may be found, for example, in U.S. Pat. No. 4,520,110 and U.S. Pat. No. 4,542,104 which are herein incorporated by reference.
Also within the scope of the present invention are antibodies conjugated to one or more prodrugs. *"Prodrug"* refers to a pharmacologically inactive derivative of a drug molecule that requires a transformation within the body to release the active drug. Typically, prodrugs are designed to overcome pharmaceutical and/or pharmacokinetically based problems associated with the parent drug molecule that would otherwise limit the clinical usefulness of the drug.
Antibodies of the invention may also be conjugated to photosensitizers, which in turn provide sensitivity to treatment with light of certain wavelength.
The first photosensitizer to receive FDA approval was Photofrin, produced by QLT Photo Therapeutics in Canada Another widely studied photosensititer is aminolevulnic acid. Other molecules in clinical trials are SnET2, which is a chlorin photosensitizer produced as Purlytin by Pharmacia/Upjohn. Another molecule is mTHPC produced by Scotia Pharmaceuticals as Foscan, which is a metatetrahydroxyphenylchlorin. Macular degeneration has been inhibited by using the photosensitizer Verteporfin, which is a haematophorphyrin derivative. These and several other molecules used as photosensitizers have been reviewed by Stewart and colleagues (Radiother Oncol 1998; 48:233) and by Dougherty and colleagues (J Natl Cancer Inst 1998; 90:889).

The above-described antibodies may be employed to isolate or to identify clones expressing the polypeptide or to purify the polypeptides by affinity chromatography. Preferably, antibodies against the polypeptides of the present invention may also be employed to treat or to diagnose neoplastic diseases.

The present invention is further illustrated by the following examples, which should not be construed as limiting in any way. The contents of all cited references (including literature references, issued patents, published patent applications), as cited throughout this application, are hereby expressly incorporated by reference. The practice of the present invention will employ, unless otherwise indicated, conventional techniques of cell biology, cell culture, molecular biology, transgenic biology, microbiology, recombinant DNA, and immunology, which are known to those skilled in the art. Such techniques are explained fully in the literature.

### EXAMPLES

### Example 1. RNA isolation from colon tissue and cDNA labelling methods.

For the diagnosis and treatment of various forms of non-steroid dependent cancers, particularly those cancers stemming from the neoplastic transformation of epithelial cells, the identification of disease-specific molecular markers is of utmost importance. A prerequisite for the successful identification of molecular-based differences between tumour tissues and those isolated from healthy individuals lies in the comparative transcriptomic analysis of differentially regulated genes in isolated epithelial cells from both healthy and tumourigenic tissues.

For this purpose, tissue samples were collected from colon cancer patients at varying stages of the disease, together with healthy colon tissue taken from a distant site (sample set). Tissue samples were collected from both male and female patients of varying ages at hospitals in Cottbus (Carl-Thiem-Khnikum Cottbus, Chirurgische Klinik, 03048 Cottbus, Germany), Magdeburg (Otto-von-Guericke-Klinik, Leipziger Strasse 44, 39120 Magdeburg, Germany) and Erlangen (Friedrich-Alexander-Universität, Chirurgische Klinik, 91023 Erlangen, Germany).

Epithelial cells were isolated according to the methods described in the patent WO98/43091 'Diagnosis of Epithelial Cell Abnormalities'.

The tissue samples were initially prepared by incubating with RNAlater™ (Ambion, UK) for 15 min. on ice. Following incubation, the tissue samples were reduced in size (ca. 1 mm) using a scalpel and mechanically separated using a steel mesh of 300µM. Following separation, cells were washed with 50ml of IX PBS, 50ml RNAlater™. (Ambion, UK), and 0.8 mM Benzamidine, 3mM EDTA, 5mg/100ml Leupeptin® and 2mM Pefabloc® (wash buffer), centrifuged for 10 minutes at 4°C at 300g and suspended in 4 ml of wash buffer. In order to isolate the epithelial cells from surrounding cells, the cell suspension was contacted with an antibody specific for epithelial cells (anti-BerEP4) covalently linked to magnetic beads. More specifically, the cell suspension was mixed with 80µl anti-BerEP4 Dynabeads® (Deutsche Dynal GmbH, Germany) and incubated for 30 min at 2-8°C in wash buffer. The epithelial cell-bound Dynabeads® were collected using a magnet (Deutsche Dynal GmbH, Germany), aliquoted on ice and stored at -80°C.

Total RNA was isolated using RNAeasy (Qiagen, Germany) according to the manufacturers instructions. RNA quality was confirmed using RNA 6000 Nano Lab Chips (Agilent, Palo Alto) and Agilent's 2100 Bioanalyzer system. Using the LabelStar™ kit (Qiagen, Germany) for cDNA labelling and cleanup, 10µg of total RNA derived from each tissue sample was reversed-transcribed to generate cyanine-3 (Cy-3) and cyanine-5 (Cy-5) labelled cDNA targets and prepared for use on microarrays according to manufacturers instructions.

In order to identify which genes are differentially expressed in tumour versus healthy tissue samples, each sample set was analysed using a microarray containing more than 12000 expressed human sequences (Human-1 cDNA Microarray, Agilent Technologies, USA). Cy-3-labelled cDNAs from epithelial cells isolated from non-tumour tissue were mixed with Cy-5-labelled cDNAs from epithelial cells isolated from tumour tissue and allowed to hybridise with the microarray at 65°C, overnight (according to manufacturer's instructions, Human-1 cDNA Microarray Kit, Agilent Technologies, USA). All hybridizations were done as fluor reversal pairs. Following hybridisation, the microarrays were washed to remove any unbound molecules and subsequently scanned for cDNAs that hybridised their complementary sequence using a dual laser microarray scanner from Agilent Technologies (Agilent Technologies, USA). Subsequently, the Feature Extraction Software (Version A6.1.1) from Agilent Technologies was used to correlate the location of the various chip features (spots) with the annotated coding sequences on the microarray. The software was also employed to calculate the signal intensity of a single chip feature in order to provide information regarding the expression level of its respective coding sequence in tumour and healthy tissue (Figure 1). Following feature extraction, data analysis was performed using the Rosetta Resolver system (Rosetta Inpharmatics. Kirkland, WA).

### Example 2. Data analysis using Rosetta Resolver (Rosetta Inpharmatics. Kirkland, WA)

The expression of genes encoding plasma membrane or plasma membrane-associated proteins was investigated using the Rosetta Resolver platform (Rosetta Inpharmatics. Kirkland, WA). Using the publicly available LocusLink database (NCBI), the gene names and database identifiers for all genes encoding known plasma membrane or plasma membrane-associated proteins were extracted can located to their corresponding feature identified on the Human-1 cDNA Microarray (Agilent Technologies, USA). At the time of the investigation, of the 1480 known plasma membrane or associated proteins entered in LocusLink (NCBI), the coding sequences of 1147 of these genes were present on the cDNA arrays used. These 1147 genes were combined in a bioset within Rosetta Resolver (Agilent Technologies, USA) and their expression in 15 colon samples (tumour and healthy) was analysed. Protein-encoding genes that were of particular interest were identified based on observed differential gene expression, wherein the difference in gene expression (fold change) in normal versus tumour tissue was at least 1.5. Furthermore, protein-encoding genes showing a large degree of confidence regarding gene expression measurements were also taken consideration. The degree of confidence was measured using a specific error model (performed by the Rosetta Resolver) that estimates the total error for a given measurement. This specific error model accounts for random errors as well as any unaccounted systematic errors, and calculates a P-value, a measure of confidence, for a given null (statistical) hypothesis. Only those genes fulfilling the above criteria (P-value of 0.01), and whose differential expression was found in 30% or more of the investigated patient samples, were selected (Table 1).

### Example 3. Detection of differential gene expression in patients for diagnostic purposes.

The expression pattern of a immunogenic membrane proteins of the invention protein can be analyzed by real-time, quantitative RT (reverse transcription)-PCR (polymerase chain reaction) (reference). Briefly, the total RNA from a minimum of 20 tumour and non-tumour pairs (isolated epithelial cells) of the tissue of interest and 3-4 different cell lines (controls for intra-assay variability) is reverse transcribed in triplicate using random hexamers and then amplified in parallel by real-time quantitative PCR in the presence of SYBR Green I using a pair of primers specific for the sequence of interest. These primers were designed using Applied Biosystems' *Primer Express 2.0* software; they should span an intron to avoid the detection of contaminating DNA, and should not be complementary to any other sequence of the human genome as detected doing a BLAST search. As an endogenous reference for the normalisation of the data (correction of sample to sample pipetting variations, etc.) 28S rRNA was used. Amplification and real-time detection and analysis of the fluorescent signals produced when SYBR Green intercalates into the double strand of the newly amplified sequences is routinely performed using the *ABI Prism 7000 Sequence Detection System* and associated software (v. 1.0) from Applied Biosystems. Values for every sample are normally expressed as Ct (threshold cycle) values, i.e. the cycle at which, once the background signal fixed, the fluorescent signal is first detected over a certain threshold. Extrapolation of each Ct value from an appropriate standard curve (plot of Ct values vs. quantity obtained by amplification of the target and reference genes from a series of known dilutions of a total RNA sample) will provide the relative amounts of said immunogenic membrane protein gene and reference genes in a particular replicate sample, while the ratio [mean±SD]_{target gene}/[mean±SD]_{reference gene} will normalise the amount of a given target within that particular sample, allowing comparison between samples. Thus, once normalised, the amount of a given target (polynucleotide sequence encoding said immunogenic membrane protein) in each tumour will be compared to the amount of the same target in the corresponding non-tumour sample to see if it is over- or under expressed in a particular sample, or if its expression does not change (see Table 2). Finally, analysis of the expression of a given target in all samples will provide information as to its global or partial (and at what frequency) over- (or under-) expressed in a certain tumour type.

**Table 2: Differential expression of immunogenic membrane proteins in tumour tissue (T) versus normal tissue (NT) from the same patient in colorectal cancer compared to non-cancerous disorders. Expression levels are expressed as fold-expression when normalized to normal tissue.**

| **Stage** | **tissue** | **CLNS1A** | **ITM1** | **ITM2B** | **LRP4** | **OPRL1** | **PTPRO** | **STOML2** | **SYPL** | **TLR3** | **TM4SF6** | **TM9SF2** | **ZP3** |
|---|---|---|---|---|---|---|---|---|---|---|---|---|---|
| Diverticulitis | C119NT | 1,00 | 1,00 | 1,00 | 1,00 | 1,00 | 1,00 | 1,00 | 1,00 | 1,00 | 1,00 | 1,00 | 1,00 |
| | C119 T | **0,52** | **0,64** | **0,39** | **0,24** | **0,47** | **0,15** | **0,58** | **0,45** | **0,19** | **0,30** | **0,50** | **0,31** |
| | C122 NT | 1,00 | 1,00 | 1,00 | 1,00 | 1,00 | 1,00 | 1,00 | 1,00 | 1,00 | 1,00 | 1,00 | 1,00 |
| | C122 T | **1,58** | **1,27** | **1,34** | **1,21** | **0,73** | **1,98** | **1,53** | **1,64** | **1,53** | **1,63** | **1,56** | **1,37** |
| Intraepith. neoplasia | B105 NT | 1,00 | 1,00 | 1,00 | 1,00 | 1,00 | 1,00 | 1,00 | 1,00 | 1,00 | 1,00 | 1,00 | 1,00 |
| | B105 T | **4,82** | **3,82** | **1,13** | **3,26** | **2,10** | **3,70** | **3,41** | **4,90** | **0,80** | **3,28** | **2,02** | **8,95** |
| UICC I | C314 NT | 1,00 | 1,00 | 1,00 | 1,00 | 1,00 | 1,00 | 1,00 | 1,00 | 1,00 | 1,00 | 1,00 | 1,00 |
| | C314 T | **3,18** | **1,54** | **0,92** | **1,95** | **2,82** | **1,58** | **2,05** | **2,33** | **0,59** | **3,18** | **1,14** | **6,03** |
| UICC II | B034 NT | 1,00 | 1,00 | 1,00 | 1,00 | 1,00 | 1,00 | 1,00 | 1,00 | 1,00 | 1,00 | 1,00 | 1,00 |
| | B034 T | **3,01** | **4,20** | **1,68** | **0,60** | **2,39** | **0,70** | **4,81** | **8,34** | **0,85** | **19,25** | **2,26** | **30,48** |
| | B089 NT | 1,00 | 1,00 | 1,00 | 1,00 | 1,00 | 1,00 | 1,00 | 1,00 | 1,00 | 1,00 | 1,00 | 1,00 |
| | B089 T | **7,75** | **8,86** | **1,78** | **10,03** | **2,98** | **11,77** | **10,20** | **9,47** | **3,61** | **20,07** | **4,38** | **43,92** |
| | C089 NT | 1,00 | 1,00 | 1,00 | 1,00 | 1,00 | 1,00 | 1,00 | 1,00 | 1,00 | 1,00 | 1,00 | 1,00 |
| | C089 T | **1,71** | **1,95** | **2,19** | **0,67** | **1,01** | **3,07** | **3,48** | **2,80** | **1,13** | **4,07** | **3,59** | **4,26** |
| | C128 NT | 1,00 | 1,00 | 1,00 | 1,00 | 1,00 | 1,00 | 1,00 | 1,00 | 1,00 | 1,00 | 1,00 | 1,00 |
| | C128 T | **1,98** | **1,74** | **1,43** | **4,08** | **1,37** | **6,35** | **2,80** | **2,40** | **1,67** | **4,40** | **2,63** | **1,43** |
| | MD135 NT | 1,00 | 1,00 | 1,00 | 1,00 | 1,00 | 1,00 | 1,00 | 1,00 | 1,00 | 1,00 | 1,00 | 1,00 |
| | MD135 T | **5,58** | **8,55** | **1,84** | **2,41** | **8,48** | **77,53** | **13,96** | **5,71** | **6,36** | **110,92** | **4,73** | **38,59** |
| UICC III | C118 NT | 1,00 | 1,00 | 1,00 | 1,00 | 1,00 | 1,00 | 1,00 | 1,00 | 1,00 | 1,00 | 1,00 | 1,00 |
| | C118T | **2,70** | **2,41** | **1,15** | **7,44** | **9,93** | **8,87** | **2,27** | **2,86** | **0,24** | **4,16** | **2,11** | **16,20** |
| | C150 NT | 1,00 | 1,00 | 1,00 | 1,00 | 1,00 | 1,00 | 1,00 | 1,00 | 1,00 | 1,00 | 1,00 | 1,00 |
| | C150 T | **2,86** | **3,90** | **1,53** | **9,50** | **2,25** | **27,83** | **3,51** | **3,34** | **1,74** | **1,83** | **2,00** | **20.75** |
| | C171 NT | 1,00 | 1,00 | 1,00 | 1,00 | 1,00 | 1,00 | 1,00 | 1,00 | 1,00 | 1,00 | 1,00 | 1,00 |
| | C171 T | **3,17** | **2,60** | **1,87** | **1,27** | **1,42** | **1,87** | **3,71** | **4,68** | **0,54** | **5,89** | **2,26** | **9,98** |
| | C310 NT | 1.00 | 1,00 | 1,00 | 1,00 | 1,00 | 1,00 | 1,00 | 1,00 | 1,00 | 1,00 | 1,00 | 1,00 |
| | C310 T | **1,95** | **1,52** | **1,69** | **0,58** | **3,21** | **1,93** | **1,45** | **1,58** | **1,19** | **1,64** | **1,83** | **3,38** |
| | MD142 NT | 1,00 | 1,00 | 1,00 | 1,00 | 1,00 | 1,00 | 1,00 | 1,00 | 1,00 | 1,00 | 1,00 | 1,00 |
| | MD142 T | **3,66** | **6,82** | **2,90** | **0,84** | **2,63** | **37,27** | **7,82** | **11,18** | **0,86** | **18,51** | **6,64** | **20,44** |
| | MD150 NT | 1,00 | 1,00 | 1,00 | 1,00 | 1,00 | 1,00 | 1,00 | 1,00 | 1,00 | 1,00 | 1,00 | 1,00 |
| | MD150 T | **4,34** | **12,38** | **3,14** | **13,24** | **30,63** | **5,68** | **6,25** | **11,67** | **0,74** | **74,03** | **5,57** | **11,31** |
| UICC IV | C111 NT | 1,00 | 1,00 | 1,00 | 1,00 | 1,00 | 1,00 | 1,00 | 1,00 | 1,00 | 1,00 | 1,00 | 1,00 |
| | C111 T | **1,78** | **2,91** | **2,17** | **0,90** | **1,76** | **0,75** | **2,98** | **2,46** | **2,23** | **3,11** | **1,90** | **3,71** |
| | MD214 NT | 1.00 | 1,00 | 1,00 | 1,00 | 1,00 | 1,00 | 1,00 | 1,00 | 1,00 | 1,00 | 1,00 | 1,00 |
| | MD214 T | **3,77** | **5,22** | **3,85** | **18,64** | **2,27** | **4,24** | **6,29** | **3,98** | **3,17** | **10,10** | **2,96** | **17,39** |

All cancer cases were identified by an at least two-fold higher expression in tumour tissue versus normal tissue of at least one of the immunogenic membrane proteins of the invention. Additionally, Fig. 2 shows the results of a diagnosis based on TM4SF6 expression.

### Example 4. Antisense suppression of protein expression

The gene expression of said immunogenic membrane proteins of the invention can be specifically downregulated in order to reduce the tumourigenic potential of cells overexpressing said protein(s). This recently developed and widely applied technology uses short (21bp) synthetic double stranded RNA duplexes (silencer RNA, siRNA) that are complementary to the coding sequence of the target gene mRNA and trigger specific mRNA degradation. Many mammalian genes have already been "knocked down" using transfection of siRNA into tumour cell lines and resulting phenotypes have been described.

Briefly, for each of the above mentioned protein genes (SYPL, STOML2, RAGA, CLNS1A, PRNP, GNB2L1, GNG4, ITM2B, ITM1, TM9SF2, TM4SF6, OPRL1, LRP4, GLEPP1, TLR3, and ZP3), 3 to 5 siRNAs, at different positions on their corresponding mRNAs, were designed using the Dharmacon "siDESIGN Center" Software as well as an additional BlastN2 in Human Unigene to minimize off-target homologies. Synthetic siRNAs were ordered from Dharmacon or MWG biotech. Different human tumour cell lines were transfected in triplicate (using Lipofectamine 2000, Invitrogen) with 50nM of each siRNA for three consecutive days and analyzed on day 4-post first transfection for gene knockdown. Analysis of the gene knockdowns is performed either by quantitative RT-PCR or - if an antibody against a given protein is available - by Western blot.
Fig 3 shows the result of a knock-down with the TM4SF6 specific siRNAs, SF1 and SF2 as quantified by RT-PCR.
In those cases where efficient gene knock-downs correspond to reduced cell proliferation, further phenotype analyses can be performed, for example, staining for apoptosis (Annexin V), cell cycle analysis (using RNAse treatment and propidium iodide staining) and a metabolic activity assay (WST-1, Roche). Alternatively, to exclude non-transfected cells from the assays, these analyses will be performed with the corresponding siRNA expression plasmids (pSilencer from Ambion or pRNA from GenScript) under G418 (Neomycin) selection. An inhibition of expression of a gene encoding an immunogenic membrane protein of the invention, will increase apoptosis, cell cycle arrest and/or reduced metabolic activity.

**Table 4: Therapeutic siRNA molecules that are able to suppress the expression of an immunogenic membrane protein of the invention**

| **PROTEIN INFORMATION** | | | | **PEPTIDE INFORMATION** | | |
|---|---|---|---|---|---|---|
| **Gene ID** | **EP Nr.** | **SwissProt ID** | **Targeted Domain** | **Position** | **Sequence** | **Length** |
| ITM1 | EP03-405 | P45977 | 9^{th} non-TM domain | 333-347 | C-LLDPSYAKNNIPIIA | 15 aa. +1 |
| | | | 14^{th} non-TM domain | 655-669 | C-KRPPGFDRVRNAEIG | 15 aa. +1 |
| ZP3 | EP03-409 | P21754 | Extracel. Domain | 175-189 | C-RLMEENWNAEKRSPT | 15 aa. +1 |
| | | | (23-387) | 368-380 | C-LDRRGDHEVEQWA | 13 aa. +1 |
| SYPL | EP03-413 | Q16563 | 1^{st} vesicular domain | 77-91 | C-TFGYPFRLNEASFQP | 15 aa. +1 |
| | | | 2^{nd} vesicular domain | 183-196 | C-GHNIIDELPPCKKK | 14 aa. +1 |
| TM9SF2 | EP03-416 | Q99805 | 1^{st} (long) lumenal | 45-59 | C-NFCDEEKKSDECKAE | 15 aa. +1 |
| | | | domain (29-300) | 102-115 | C-GERTEPSPYKFTFN | 14 aa. +1 |

### Example 5. Identification of antibodies specific for immunogenic membrane proteins of the invention.

Based at least in part on the results described in the previous examples, a therapeutic agent will interact with said immunogenic membrane protein and can therefore be used for treating non-steroid dependent cancers. Specific antibodies are widely used as agents interacting with a given molecule.

A soluble binding fragment of said protein was prepared by expressing a TM4SF6 encoding gene fragment in *E*. *coli*.

### Cloning of the EC2-loop of TM4SF6:

The DNA sequence of interest was amplified using pfu polymerase and the primers EC2-a (ggatccagacatgagattaagaac) and EC2-b (aagcttattctgactctataatgg) from a sequence verified clone of the gene TM4SF6. The PCR product was subcloned into the vector pCR2.1 using the TA cloning strategy. The DNA sequence of interested was cloned into the bacterial expression plasmid PQE-30 from Qiagen using the restriction enzymes BamBI (in primer EC2-a) and Hind III (in primer EC2-b) creating the expression plasmid pQE-415-EC2. After sequence verification the vector was transformed into BL21 (DE3).

### TM4SF6 Expression:

An over-night culture of pQE-415-EC2 in BL21 (Amp.) was grown at 30°C in LB-NZ Medium (5g NaCl, 5g yeast extract, 10g NZ-Amine), inoculated 450ml LB-NZ (in 11 flask) the next moming(to an OD₆₀₀ of 0,05 - 0,1) and grown at 37°C to an OD600 of 0,5 - 0,6.

The culture was induced with 5mM (final concentration) IPTG; after 5 - 6 h the cells were harvested, aliquoted in 3 tubes, washed with 20mM phosphate buffer pH8 and stored at -80°C for further use.

### Preparation of cell extract:

The cells were thawed and 30ml 20mM phosphate buffer pH8 added; lysozyme to a final concentration of Img/ml, and the mixture incubated by gentle shaking for 30 min. on ice, followed by addition of urea to a final concentration of 5M. The DNA was disrupted by sonication and the extract was centrifuged at 25.000 rpm using a MLA-80 ultracentrifuge rotor for 30 min.

### Column Chromatography using the ÄKTA FPLC:

1st run: After the supernatant was loaded onto a Ni-Agarose column from Sigma the column was washed with 5 column volumes of 100% buffer A1 (20mM Phosphate Puffer pH8, 200mM NaCl, 5M Harnstoff) and then with 5 column volumes of buffer B1 (20mM Phosphate Puffer pH8, 200 NaCl, 5M Harnstoff, 1M Imidazol). The protein was eluted with 100% buffer B1.
2nd run: For rechromatography the fractions of interest was diluted 1:10 using buffer A2 (20mM Phosphate Puffer pH6,3, 250 mM NaCl). The new Ni-Agarose column was washed with buffer B2 (20mM Phosphate Puffer pH6,3, 250mM NaCl, 1M Imidazol) in a gradient of 0 - 30 % in 10 column volumes. The protein was eluted with 100 % buffer B2.
Chromatography using Resource S: The fraction of interest from the 2nd run was diluted 1:20 in buffer A3 (20mM MES, pH6) and loaded onto a 1ml Respurce S column. The proteins were eluted using a 0 - 100 % gradient of buffer B3 (20mM MES, 500mM NaCl, pH6 in 30 min (flow rate 1ml/min).
Figure 4 shows an SDS-PAGE stained with Coomassie Blue of the purified fractions.

Additionally, rabbits were immunized with synthesised peptides spanning certain domains according to table 5:

**Table 5: Peptides used for immunization of rabbits**

| **Gene** | **Targeted Domain** | **Position (aa)** |
|---|---|---|
| TM4SF6 | 2^{nd} (large) extracel. domain (115-208) [EC2 loop] | 122-131 |
| | | 187-195 |
| | | 136-148 |
| | | 183-196 |
| ITM1 | 9^{th} non-TM domain | 333-347 |
| | 14^{th} non-TM domain | 655-669 |
| ZP3 | Extracel. Domain (23-387) | 175-189 |
| | | 368-380 |
| SYPL | 1^{st} vesicular domain | 77-91 |
| | 2^{nd} vesicular domain | 183-196 |
| TM9SF2 | 1^{st} (long) lumenal domain (29-300) | 45-59 |
| | | 102-115 |

### KLH coupling

100mg of keyhole limpet hemocyanin (KLH) were dissolved in 2ml water and dialyzed against 21 of 0.1M NaPhoshate pH 7.8 overnight. This is to remove any contaminating thiols or amino compounds. Aggregates were removed by centrifugation for 10 minutes at full speed in microfuge.
For -NH2 coupling, 5mg peptide were added to one half of the KLH, followed by glutaraldehyde to 0.1% final concentration and the pH was adjusted to 7.8 if necessary using NaOH. Incubation was 8-12 hrs at 4 degrees, rotating gently.
After adding a tiny pinch of NaBH4 the mixtures was incubated 8-12 hrs at 4 degrees.
For the -SH coupling, the other aliquot of KLH was warmed to room temp. 1/9 th volume of Iodoacetic acid N-hydroxysuccinimide ester (Sigma) at 100mg/ml in DMSO was added. After 10 minutes at room temp the KLH will start to get a little cloudy. It was purified on a P-10 column equilibrated with 0.1M NaPhosphate pH 7.8. The KLH containing fractions were pooled by color, 5mg of peptide added, and incubated at least 8 hrs at 4 degrees, rotating gently.

The rabbits were immunized with 450-600µg of KLH-conjugated peptide on day 0, 14 and 28 with complete Freunds adjuvant for the primary injection and incomplete adjuvant for the booster injections.

### Purification of antibodies

The respective peptides were coupled to AF-Amino Toyopearl 650 M according to the manufacturers instructions and used for batch chromatography in PBS, followed by 5 washing steps with PBS. Antibodies specifically bound to the matrix were eluted with 100 mM glycine pH 2.5.
Purified antibodies were stored in PBS/ 0.01% sodium azide/ 1% BSA/50% glycerol at -20°C.

### Example 6: Immunohistochemistry of tissue sections

Immunohistochemistry is an *in vitro* diagnostic method well known in the art. The following protocol was used to stain paraffin embedded tissue section for TM4SF6 using the EP03-415 antibody.

| | |
|---|---|
| Pre-incubation: | TBS/0.1%Triton -X-100 (30 min); rt |
| POX-Inhibition: | 3% H₂O₂ (10 min) |
| Inhibition: | SEA-Block (10 min) |
| 1st Ab Incubation: | 1:200 dilution in TBS/10% SEA-Block (30 min); rt |
| Washing: | TBS/0.05% Tween 20 (3x 10 min); rt |
| 2nd Ab and Development: | En Vision, POD (DAKO); 30 min; rt |

| | |
|---|---|
| DAB; 5 min; rt Fig. 5 shows the immunohistochemistry results from patients suffering from colon cancer and melanoma. The staining pattern allows a distinction between healthy, non-transformed tissue and cancerous cells. thus, immunohistochemistry can be employed for diagnostic purposes using biopsies and/or surgery samples. | |

The present invention relates to a method of screening for a therapeutic agent for the treatment of_a non-steroid dependent cancer charcterized by the aberrant expression and/or an altered biological activity of at least one immunogenic membrane protein selected from the group consisting of TM4SF6, SYPL, STOML2, RAGA, CLNS1A, PRNP, GNB2L1, GNG4, TTM2B, ITM1, TM9SF2, OPRL1, LRP4, GLEPP1, TLR3, or ZP3 polypeptide and/or fragment(s) thereof.
In a preferred embodiment the method of screening for a therapeutic agent according to the invention comprises a) contacting a reporter construct under the control of a promoter of said immunogenic membrane protein with a test molecule or compound, or a library of test molecules or compounds, under conditions to allow specific binding and/or interaction and b) detecting the level of expression of the reporter construct, wherein an alteration in the level of expression relative to a control indicates a potential therapeutic activity.

In another preferred embodiment of the screening method according to the invention the method comprises a) contacting said at least one immunogenic membrane protein, derivative or fragment(s) thereof with a test molecule or compound, or a library of test molecules or compounds, under conditions to allow specific binding and/or interaction and b)detecting the level of specific binding and/or interaction, wherein an alteration in the level of interaction relative to a control indicates a potential therapeutic activity.

In the screening method of the invention the screened therapeutic agent preferably suppresses the expression and/or the biological activity of said at least one immunogenic membrane protein. Preferably the library of test molecules or compounds used in the method according to the invention is selected from the group consisting of DNA and/or RNA molecules, peptides, agonists, antagonists, monoclonal antibodies, immunoglobulins, small molecule drugs, pharmaceutical agents and combinations thereof.

It is another object of the present invention to provide a pharmaceutical composition comprising a therapeutic agent identified by the method of screening according to the invention, or a fragment, derivative or homologue thereof. In a preferred embodiment the pharmaceutical composition further comprises a pharmaceutically acceptable carrier.

The present invention further relates to the use of a therapeutic agent identified by the screening method according to the invention or to the use of a derivative or homologue thereof or of a delivery complex containing and/or expressing said therapeutic agent or a derivative or homologue thereof for the preparation of a pharmaceutical composition for the treatment of a non-steroid dependent cancer. The present invention further relates to the use

It is another object of the present invention to provide a method for the suppression of a polynucleotide sequence encoding said at least one immunogenic membrane protein for the purpose of modulating proliferation and/or differentiation or cell death of target cells comprising a) providing a pharmaceutical composition according to the invention or a composition that comprises a therapeutic agent identified by the method of screening according to the invention and b) contacting a therapeutically effective amount of the composition from a) with said target cells. In a preferred embodiment said target cells are neoplastic cells.

The present invention further relates to a method for treating a non-steroid dependent cancer resulting from the aberrant expression and/or from an altered biological activity of said at least one immunogenic membrane protein comprising administering to a subject a therapeutically effective amount of a pharmaceutical composition according to the invention or or a therapeutic agent identified by the method according to the invention.

In a preferred embodiment of the invention the therapeutic agent used in the method for the suppression of a polynucleotide sequence or in the method for treating a non-steroid dependent cancer is an antisense polynucleotide sequence complementary to said at least one immunogenic membrane protein or a fragment thereof. Preferably the binding of said antisense polynucleotide sequence is effective in altering transcription or translation of an mRNA encoding said at least one immunogenic membrane protein according to the invention or a fragment thereof in a host cell expressing said mRNA. Preferably the antisense polynucleotide sequence is expressed from a viral vector, in particular a vaccinia virus, a retrovirus, an adenovirus, or a combination thereof. In another preferred embodiment the antisense polynucleotide sequence is in a delivery complex. The delivery complex according to the invention is preferably a sterol, a lipid or a virus.

It is another object of the present invention to provide a method for the treatment of non-steroid dependent cancer by expressing said at least one immunogenic membrane protein according to the invention or a fragment thereof comprising the stepps of a) isolating a cell or cells, b) contacting said cell(s) with an antisense molecule as defined above and c) delivering said cell(s) to a subject suffering from non-steroid dependent cancer.

The present invention further relates to the use of an antisense molecule according to the invention or of a cell expressing and/or containing said antisense molecule for the preparation of a pharmaceutical composition for the treatment of a non-steroid dependent cancer. In a preferred embodiment of the method for treatment of non-steroid dependent cander or of the use of an antisense molecule said cell(s) are derived from the subject_to_be treated.

The present invention further relates to the use of at least one of said immunogenic membrane proteins, fragments, derivatives or homologues thereof, or of a cell containing and/or expressing at least one of said immunogenic membrane proteins or fragments, derivatives or homologues thereof, for the preparation of a pharmaceutical composition for the vaccination of subjects.

The present invention further relates to a method of screening for an agent binding specifically to a polynucleotide encoding said at least one immunogenic membrane polypeptide or to said at least one immunogenic membrane polypeptide comprising a) contacting said at least one immunogenic membrane protein, derivative or fragment(s) thereof with a test molecule or compound, or a library of test molecules or compounds, under conditions to allow specific binding and/or interaction and b) detecting the level of specific binding and/or interaction. In a preferred embodiment of the invention the library of test molecules or compounds is selected from the group consisting of DNA and/or RNA molecules, peptides, agonists, antagonists, monoclonal antibodies, immunoglobulins, small molecules, pharmaceutical agents and combinations thereof.

It is another object of the present invention to provide an agent binding specifically to a polynucleotide encoding said at least one immunogenic membrane polypeptide or to said at least one immunogenic membrane polypeptide according to the invention. Preferably said agent is an antibody, in particular a monoclonal antibody. In a preferred embodiment said agent or satd antibody comprise an additional moiety.

The present invention further relates to the use of an agent or an antibody according to the invention for the preparation of a pharmaceutical composition for the treatment, diagnosis or the visualisation of non-steroid dependent cancer.

According to the invention the non-steroid dependant cancer is preferably cancer of the breast, lung, gastrointestinal, prostate, ovar, cervix, endometrium, bladder, skin, and/or other cancers arising from epithelial tissue. More preferred the non-steroid dependant cancer according to the invention is a cancer of the skin, in particular melanoma, or colorectal cancer.

It is a further object of the invention to provide a method for determining whether a subject is at risk of developing or has a non-steroid dependent cancer comprising a) determining the amount of polynucleotides encoding said at least one immunogenic membrane polypeptide or the amount of said immunogenic membrane polypeptide(s) according to the invention in a sample of said subject and b) comparing said polynucleotide or polypeptide amount with a control amount that is representative of a healthy subject.

The present invention further relates to a kit for identifying a subject at risk of developing or has a non-steroid dependent cancer caused by the aberrant expression of a polynucleotide sequences encoding a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide comprising, the means for measuring the level of a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3-encoding polynucleotide sequence (X68194, AF190167, BC006433, X91788, M13667, BC000214, U31382, NM 021999, L38961, U81006, AF043906, U30185, AB011540, U20489, U88879, X56777, respectively) in a sample of cells of said subject and instructions to use the kit.

The present invention further relates to a kit for identifying a subject at risk of developing or has a non-steroid dependent cancer resulting from an altered biological activity of an SYPL. STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide comprising, the means for measuring the level of a SYPL, STOML2, RAGA, CLNS1A, PRNP, GNB2L1, GNG4, ITM2B, ITM1, TM9SF2, TM4SF6, OPRL1, LRP4, GLEPP1, TLR3, or ZP3 polypeptide in a sample of cells of said subject and instructions to use the kit.
According to the invention the at least one immunogenic membrane peptide is preferably of human origin.

## Claims

1. An agent binding specifically to an immunogenic membrane polypeptide selected from the group consisting of TM9SF2 and LRP4 or to a polynucleotide encoding said immunogenic membrane polypeptide.

2. The agent of claim 1, which is an antibody, preferably a monoclonal antibody.

3. The agent of claim 1 or the antibody of claim 2, comprising an additional moiety.

4. The agent of claim 1, wherein said agent is an antisense polynucleotide sequence complementary to said polynucleotide encoding said immunogenic membrane protein or a fragment thereof.

5. Use of an agent or antibody of any one of claims 1 to 4 for the preparation of a pharmaceutical composition for the treatment, diagnosis or the visualisation of non-steroid dependent cancer.

6. The use of claim 5, wherein the non-steroid dependant cancer is cancer of the breast, lung, gastrointestinal, prostate, ovar, cervix, endometrium, bladder, skin, and/or other cancers arising from epithelial tissue.

7. The use of claim 6, wherein the non-steroid dependant cancer is cancer of the skin, in particular melanoma, or colorectal cancer.

8. Use of an antisense molecule as defined in claim 4 or of a cell expressing and/or containing said antisense molecule for the preparation of a pharmaceutical composition for the treatment of a non-steroid dependent cancer.

9. The use of claim 8, wherein said cell(s) are derived from the subject to be treated.

10. Use of an immunogenic membrane protein selected from the group consisting of TM9SF2 and LRP4, or a fragment, derivative or homologue thereof, or of a cell containing and/or expressing said immunogenic membrane protein, or fragment, derivative or homologue thereof, for the preparation of a pharmaceutical composition for the vaccination of subjects.

11. A method of screening for a therapeutic agent for the treatment of a non-steroid dependent cancer **characterised by** the aberrant expression and/or an altered biological activity of at least one immunogenic membrane protein selected from the group consisting of TM9SF2 and LRP4 polypeptide and/or fragment(s) thereof, said method comprising:
a. contacting a reporter construct under the control of a promoter of said immunogenic membrane protein with a test molecule or compound, or a library of test molecules or compounds, under conditions to allow specific binding and/or interaction; and
b. detecting the level of expression of the reporter construct,
wherein an alteration in the level of expression relative to a control indicates a potential therapeutic activity.

12. A method of screening for a therapeutic agent for the treatment of a non-steroid dependent cancer **characterised by** the aberrant expression and/or an altered biological activity of at least one immunogenic membrane protein selected from the group consisting of TM9SF2 and LRP4 polypeptide and/or fragment(s) thereof, said method comprising:
a. contacting said at least one immunogenic membrane protein, derivative or fragment(s) thereof with a test molecule or compound, or a library of test molecules or compounds, under conditions to allow specific binding and/or interaction; and
b. detecting the level of specific binding and/or interaction,
wherein an alteration in the level of interaction relative to a control indicates a potential therapeutic activity.

13. The method of claim 11 or 12, wherein the library of test molecules or compounds is selected from the group consisting of DNA and/or RNA molecules, peptides, agonists, antagonists, monoclonal antibodies, immunoglobulins, small molecule drugs, pharmaceutical agents and combinations thereof.

14. A pharmaceutical composition comprising a therapeutic agent identified by a method of any one of claims 11 to 13, or a fragment, derivative or homologue thereof.

15. The pharmaceutical composition of claim 14, further comprising a pharmaceutically acceptable carrier.

16. Use of a therapeutic agent identified by a method of any one claims 11 to 13, or a derivative or homologue thereof, or of a delivery complex, containing and/or expressing said therapeutic agent or a derivative or homologue thereof, for the preparation of a pharmaceutical composition for the treatment of a non-steroid dependent cancer.

17. A method of screening for an agent binding specifically to an immunogenic membrane polypeptide selected from the group consisting of TM9SF2 and LRP4, said method comprising:
a. contacting said immunogenic membrane protein, or a derivative or fragment(s) thereof with a test molecule or compound, or a library of test molecules or compounds, under conditions to allow specific binding and/or interaction; and
b. detecting the level of specific binding and/or interaction

18. The method of claim 17, wherein the library of test molecules or compounds is selected from the group consisting of DNA and/or RNA molecules, peptides, agonists, antagonists, monoclonal antibodies, immunoglobulins, small molecules, pharmaceutical agents and combinations thereof

19. A method for determining whether a subject is at risk of developing or has a non-steroid dependent cancer comprising:
a. determining the amount of polynucleotides encoding an immunogenic membrane polypeptide selected from the group consisting of TM9SF2 and LRP4, or the amount of said immunogenic membrane polypeptide(s) in a sample of said subject, and
b. comparing said polynucleotide or polypeptide amount with a control amount that is representative of a healthy subject.

20. A kit for identifying a subject at risk of developing or has a non-steroid dependent cancer caused by the aberrant expression of a polynucleotide sequence encoding a TM9SF2 or LRP4 polypeptide, said kit comprising a means for measuring the level of a TM9SF2 or LRP4-encoding polynucleotide sequence (AF043906 or AB011540, respectively) in a sample of cells of said subject and instructions to use the kit.

21. A kit for identifying a subject at risk of developing or has a non-steroid dependent cancer caused resulting from an altered biological activity of a TM9SF2 or LRP4 polypeptide, said kit comprising a means for measuring the level of a TM9SF2 or LRP4 polypeptide in a sample of cells of said subject and instructions to use the kit.
